(19) Europäisches Patentamt
European Patent Office
Office européen des brevets

(11) EP 4 745 145 A1

(12) **EUROPEAN PATENT APPLICATION**
published in accordance with Art. 153(4) EPC

(43) Date of publication:
**20.05.2026 Bulletin 2026/21**

(21) Application number: **24838870.4**

(22) Date of filing: **11.07.2024**

(51) International Patent Classification (IPC):
***C07D 513/04*** *(2006.01)* ***C07D 401/14*** *(2006.01)*
***A61K 31/4985*** *(2006.01)* ***A61K 31/429*** *(2006.01)*
***A61P 35/00*** *(2006.01)*

(52) Cooperative Patent Classification (CPC):
**A61K 31/429; A61K 31/4985; A61P 35/00; C07D 401/14; C07D 513/04**

(86) International application number:
**PCT/CN2024/104980**

(87) International publication number:
**WO 2025/011618 (16.01.2025 Gazette 2025/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.07.2023 CN 202310852344**

(71) Applicant: **Simcere Zaiming Pharmaceutical Co., Ltd.**
**Haikou, Hainan 570311 (CN)**

(72) Inventors:
- **LI, Xingming**
  **Shanghai 201318 (CN)**
- **LIU, Chengxiang**
  **Shanghai 201318 (CN)**
- **HU, Yue**
  **Shanghai 201318 (CN)**
- **XIA, Zihua**
  **Shanghai 201318 (CN)**
- **LI, Zhen**
  **Shanghai 201318 (CN)**

(74) Representative: **V.O.**
**P.O. Box 87930**
**2508 DH Den Haag (NL)**

(54) **SALT OF THIAZOLOPYRAZINE COMPOUND OR CRYSTAL THEREOF AND USE THEREOF**

(57) Provided are a pharmaceutically acceptable salt of a thiazopyrazine compound represented by formula (I) or a solvate (including a hydrate) of the pharmaceutically acceptable salt, a crystal form of the pharmaceutically acceptable salt or a crystal form of the solvate (including a hydrate) of the pharmaceutically acceptable salt, a crystal form of the compound of formula (I), a preparation method therefor, a pharmaceutical composition thereof, and the medical use thereof.

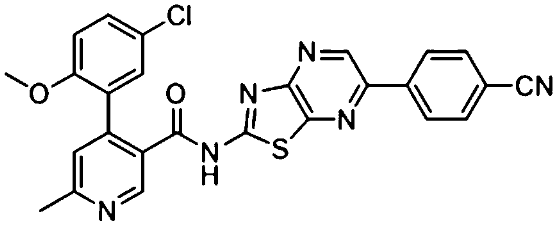

(I)

EP 4 745 145 A1

## Description

### CROSS-REFERENCE TO RELATED APPLICATIONS

The present disclosure claims priority to and the benefit of the Chinese Patent Application No. 202310852344.4, filed with the China National Intellectual Property Administration on July 12, 2023. The patent application described above is incorporated herein by reference in its entirety.

### TECHNICAL FIELD

The present disclosure relates to the field of medicinal chemistry, and in particular, to a crystal form of a thiazolopyrazine compound, a pharmaceutically acceptable salt of the thiazolopyrazine compound or a solvate (including a hydrate) of the pharmaceutically acceptable salt, a crystal form of the pharmaceutically acceptable salt or the solvate (including a hydrate) of the pharmaceutically acceptable salt, a preparation method therefor, a pharmaceutical composition comprising the same, and use thereof.

### BACKGROUND

DNA double-strand break repair is essential for maintaining genomic stability and cell survival. There are three main repair pathways for DNA double-strand breaks: homologous recombination (HR), non-homologous end joining (NHEJ), and alternative non-homologous end joining (alt-NHEJ). Microhomology-mediated end joining (MMEJ) is the most common non-traditional non-homologous end joining. Homologous recombination is a high-fidelity, error-free repair mechanism that maintains genome stability and avoids cancer induction. Non-homologous end joining and microhomology-mediated end joining are error-prone repair pathways that result in a mutation at the repair site.

Unlike normal cells, the survival of tumor cells often depends on the misregulation of DNA double-strand break repair. At the same time, aberrant DNA double-strand break repair can make tumor cells more sensitive to specific types of DNA damage. Thus, defects in DNA double-strand break repair can be exploited to develop targeted tumor therapies. Tumor cells with impaired homologous recombination or non-homologous end joining repair will be more dependent on microhomology-mediated end joining repair. Multiple lines of evidence in genetics, cell biology, and biochemistry indicate that DNA polymerase $\theta$ (POLQ or POL$\theta$) is a key protein in the process of microhomology-mediated end joining repair (Kent et al. Nature Structural & Molecular Biology (2015), 22(3), 230-237, Mateos-Gomez et al. Nature (2015), 518(7538), 254-257).

POLQ is a multifunctional enzyme composed of an N-terminal helicase domain (SF2 HEL308-type) and a C-terminal low-fidelity DNA polymerase domain (A-type) (Wood & Doublie DNA Repair (2016), 44, 22-32). The helicase domain mediates the removal of the RPA protein from single-stranded DNA and promotes annealing, the polymerase domain can extend single-stranded DNA ends and fill gaps, and the two domains work together to function in a microhomology-mediated end joining repair process.

Studies have shown that POLQ is essential for homologous recombination-deficient cells (e.g., synthetically lethal with FA/BRCA deficiency) and that the protein level of POLQ is upregulated in homologous recombination-deficient tumor cells (Ceccaldi et al. Nature (2015), 518(7538), 258-262). *In vivo* study results also show that POLQ is overexpressed in a series of homologous recombination-deficient ovarian, uterine, and breast cancers with poor prognosis (Higgins et al. Oncotarget (2010), 1, 175-184; Lemee et al. PNAS (2010), 107(30), 13390-13395; Ceccaldi et al. (2015), supra). More importantly, POLQ expression is inhibited in normal tissues compared to tumor tissues (Kawamura et al., International Journal of Cancer (2004), 109(1), 9-16).

In summary, POLQ is essential for homologous recombination-deficient cells, and there is an unmet market need for the treatment of homologous recombination-deficient tumors. Inhibition of the POLQ function can inhibit microhomology-mediated end joining repair in cells, and the development of POLQ function inhibitors can provide a novel strategy for a targeted therapy of homologous recombination-deficient tumors.

### SUMMARY

In one aspect, the present disclosure provides a pharmaceutically acceptable salt of a compound of formula I (4-(5-chloro-2-methoxyphenyl)-N-[6-(4-cyanophenyl)thiazolo[4,5-b]pyrazin-2-yl]-6-methylnicotinamide), which is a POLQ inhibitor, wherein the pharmaceutically acceptable salt is selected from a sodium salt or a potassium salt,

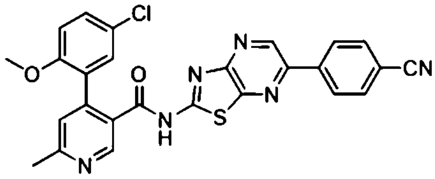

I.

In some embodiments, the present disclosure provides a pharmaceutically acceptable salt of a compound of formula I, wherein the pharmaceutically acceptable salt is a sodium salt,

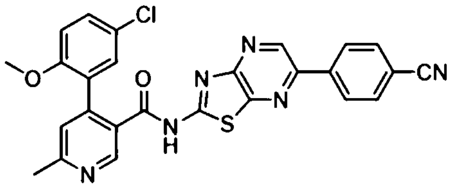

I.

In some embodiments, in the pharmaceutically acceptable salt of the compound of formula I, the molar ratio of the compound of formula I to sodium ions or potassium ions is about 1:1.

In some embodiments, in the pharmaceutically acceptable salt of the compound of formula I, the molar ratio of the compound of formula I to sodium ions or potassium ions is 1:1.

In some embodiments, the present disclosure provides a sodium salt of the compound of formula I, and in the sodium salt, the molar ratio of the compound of formula I to sodium ions is about 1:1.

In some embodiments, the present disclosure provides a sodium salt of the compound of formula I, and in the sodium salt, the molar ratio of the compound of formula I to sodium ions is 1:1.

In some embodiments, the present disclosure provides a potassium salt of the compound of formula I, and in the potassium salt, the molar ratio of the compound of formula I to potassium ions is about 1:1.

In some embodiments, the present disclosure provides a potassium salt of the compound of formula I, and in the potassium salt, the molar ratio of the compound of formula I to potassium ions is 1:1.

In some embodiments, the pharmaceutically acceptable salt of the compound of formula I may exist in an unsolvated or solvated form.

In another aspect, the present disclosure also provides a solid form of the pharmaceutically acceptable salt of the compound of formula I.

In some embodiments, the solid form of the pharmaceutically acceptable salt of the compound of formula I is selected from an amorphous form or a crystal form.

In another aspect, the present disclosure also provides a solid form of the sodium salt of the compound of formula I.

In some embodiments, the solid form of the sodium salt of the compound of formula I is selected from an amorphous form or a crystal form.

In another aspect, the present disclosure also provides a solid form of the potassium salt of the compound of formula I.

In some embodiments, the solid form of the potassium salt of the compound of formula I is selected from an amorphous form or a crystal form.

In yet another aspect, the present disclosure also provides a preparation method for the sodium salt of the compound of formula I, which comprises the step of forming a salt with the compound of formula I and a sodium-containing base.

In some embodiments, the preparation method for the sodium salt of the compound of formula I comprises reacting the compound of formula I with a sodium-containing base in a solvent to form a salt, and the solvent is selected from one or more of an alcohol solvent, DMSO, NMP, DMF, or DMA.

In some embodiments, the solvent is selected from one or more of ethanol, DMSO, NMP, DMF, or DMA.

In some embodiments, the sodium-containing base is selected from sodium tert-butoxide, sodium ethoxide, sodium methoxide, or sodium hydroxide.

In some embodiments, the sodium-containing base is selected from sodium ethoxide, sodium methoxide, or sodium hydroxide.

In some embodiments, the sodium-containing base is selected from sodium ethoxide or sodium hydroxide.

In yet another aspect, the present disclosure also provides a preparation method for the potassium salt of the compound of formula I, which comprises the step of forming a salt with the compound of formula I and a potassium-containing base.

In some embodiments, the preparation method for the potassium salt of the compound of formula I comprises reacting the compound of formula I with a potassium-containing base in a solvent to form a salt, and the solvent is selected from one or more of DMF, DMA, or NMP.

In some embodiments, the potassium-containing base is potassium hydroxide.

In yet another aspect, the present disclosure provides a solvate of the sodium salt of the compound of formula I.

In some embodiments, the solvate of the sodium salt of the compound of formula I is selected from a hydrate or an ethanolate.

In some embodiments, in the solvate of the sodium salt of the compound of formula I, the molar ratio of the sodium salt of the compound of formula I to the solvent in the solvate is about 1:1.

In some embodiments, in the solvate of the sodium salt of the compound of formula I, the molar ratio of the sodium salt of the compound of formula I to the solvent in the solvate is 1:1.

The present disclosure also provides a solid form of the solvate of the sodium salt of the compound of formula I. In some embodiments, the solid form of the solvate of the sodium salt of the compound of formula I is selected from an amorphous form or a crystal form.

In some embodiments, the solvate of the sodium salt of the compound of formula I is a hydrate.

In some embodiments, the solvate of the sodium salt of the compound of formula I is a monohydrate.

In some embodiments, the solvate of the sodium salt of the compound of formula I is an ethanolate.

In some embodiments, the solvate of the sodium salt of the compound of formula I is a monoethanolate.

In yet another aspect, the present disclosure provides a solvate of the potassium salt of the compound of formula I. In some embodiments, the solvate of the potassium salt of the compound of formula I is a hydrate.

In some embodiments, the solvate of the potassium salt of the compound of formula I is a monohydrate.

In some embodiments, in the solvate of the potassium salt of the compound of formula I, the molar ratio of the potassium salt of the compound of formula I to the solvent in the solvate is about 1:1.

In some embodiments, in the solvate of the potassium salt of the compound of formula I, the molar ratio of the potassium salt of the compound of formula I to the solvent in the solvate is 1:1.

The present disclosure also provides a solid form of the solvate of the potassium salt of the compound of formula I. In some embodiments, the solid form of the solvate of the potassium salt of the compound of formula I is selected from an amorphous form or a crystal form.

In further another aspect, the present disclosure provides a crystal form of the sodium salt of the compound of formula I.

In some embodiments, the present disclosure also provides crystal form A of the sodium salt of the compound of formula I, and the X-ray powder diffraction pattern of crystal form A, expressed in terms of diffraction angle $2\theta$, has diffraction peaks at 6.39±0.20°, 7.84±0.20°, 13.61±0.20°, and 25.46±0.20°.

In some embodiments, the X-ray powder diffraction pattern of crystal form A of the sodium salt of the compound of formula I, expressed in terms of diffraction angle $2\theta$, has diffraction peaks at 6.39±0.20°, 7.84±0.20°, 13.61±0.20°, 15.95 ±0.20°, 17.16±0.20°, 20.90±0.20°, 21.81±0.20°, 25.46±0.20°, and 30.79±0.20°.

In some embodiments, the X-ray powder diffraction pattern of crystal form A of the sodium salt of the compound of formula I, expressed in terms of diffraction angle $2\theta$, has diffraction peaks at 6.13±0.20°, 6.39±0.20°, 6.87±0.20°, 7.84 ±0.20°, 10.80±0.20°, 13.61±0.20°, 14.10±0.20°, 15.95±0.20°, 16.44±0.20°, 17.16±0.20°, 17.74±0.20°, 19.09 ±0.20°, 20.90±0.20°, 21.81±0.20°, 22.01±0.20°, 22.73±0.20°, 22.93±0.20°, 25.46±0.20°, and 30.79±0.20°. In some embodiments, the X-ray powder diffraction pattern of crystal form A of the sodium salt of the compound of formula I, expressed in terms of diffraction angle $2\theta$, is substantially as shown in FIG. 1.

In some embodiments, crystal form A of the sodium salt of the compound of formula I has a DSC spectrum with a peak at 342.84±5.0 °C. In some embodiments, the DSC spectrum of crystal form A of the sodium salt of the compound of formula I is as shown in FIG. 2.

In further another aspect, the present disclosure also provides a method for preparing crystal form A of the sodium salt of the compound of formula I, which comprises: (1) mixing the compound of formula I with absolute ethanol, and (2) adding a mixed solution of sodium hydroxide and absolute ethanol, stirring, and separating.

In some embodiments, in the method for preparing crystal form A of the sodium salt of the compound of formula I, the molar ratio of the compound of formula I to sodium hydroxide is 1:(0.5-1.5), 1:(0.9-1.2), or about 1:1.

In some embodiments, in the method for preparing crystal form A of the sodium salt of the compound of formula I, the volume (mL) of absolute ethanol is 20-45 times or 35-45 times the mass (g) of the compound of formula I in step (1).

In some embodiments, in the method for preparing crystal form A of the sodium salt of the compound of formula I, the stirring temperature is 20 °C-60 °C or 20 °C-30 °C in step (2).

In further another aspect, the present disclosure provides a crystal form of the compound of formula I.

In some embodiments, the present disclosure also provides crystal form B of the compound of formula I, and the X-ray powder diffraction pattern of crystal form B, expressed in terms of diffraction angle $2\theta$, has diffraction peaks at 12.10 ±0.20°, 17.79±0.20°, 20.13±0.20°, and 25.47±0.20°.

In some embodiments, the X-ray powder diffraction pattern of crystal form B of the compound of formula I, expressed in terms of diffraction angle $2\theta$, has diffraction peaks at 7.98±0.20°, 12.10±0.20°, 14.59±0.20°, 17.80±0.20°, 20.13±0.20°, 22.51±0.20°, 23.61±0.20°, 25.47±0.20°, 26.74±0.20°, and 27.66±0.20°.

In some embodiments, the X-ray powder diffraction pattern of crystal form B of the compound of formula I, expressed in terms of diffraction angle $2\theta$, has diffraction peaks at 7.98±0.20°, 12.10±0.20°, 14.59±0.20°, 15.97±0.20°, 17.31±0.20°, 17.79::í:0.20°, 18.23±0.20°, 20.13±0.20°, 22.51±0.20°, 23.31±0.20°, 23.61±0.20°, 23.98±0.20°, 24.56 ±0.20°, 25.47±0.20°, 26.74±0.20°, and 27.66±0.20°.

In some embodiments, the X-ray powder diffraction pattern of crystal form B of the compound of formula I, expressed in terms of diffraction angle $2\theta$, is substantially as shown in FIG. 4.

In some embodiments, crystal form B of the compound of formula I has a DSC spectrum with a peak at 343.98 ±5.0 °C. In some embodiments, the DSC spectrum of crystal form B of the compound of formula I is as shown in FIG. 5.

In further another aspect, the present disclosure provides a crystal form of the monoethanolate of the sodium salt of the compound of formula I.

In some embodiments, the present disclosure also provides crystal form C of the monoethanolate of the sodium salt of the compound of formula I, and the X-ray powder diffraction pattern of crystal form C, expressed in terms of diffraction angle $2\theta$, has diffraction peaks at 17.14±0.20°, 21.85±0.20°, 25.46±0.20°, and 25.74±0.20°.

In some embodiments, the X-ray powder diffraction pattern of crystal form C of the monoethanolate of the sodium salt of the compound of formula I, expressed in terms of diffraction angle $2\theta$, has diffraction peaks at 12.37±0.20°, 17.14 ±0.20°, 20.07±0.20°, 21.40±0.20°, 21.85±0.20°, 23.39±0.20°, 24.51±0.20°, 24.99±0.20°, 25.46±0.20°, and 25.74 ±0.20°.

In some embodiments, the X-ray powder diffraction pattern of crystal form C of the monoethanolate of the sodium salt of the compound of formula I, expressed in terms of diffraction angle $2\theta$, has diffraction peaks at 12.37±0.20°, 12.70 ±0.20°, 15.47±0.20°, 17.14±0.20°, 17.96±0.20°, 19.76±0.20°, 20.07±0.20°, 20.30±0.20°, 21.40±0.20°, 21.85 ±0.20°, 22.29±0.20°, 23.39±0.20°, 24.51±0.20°, 24.99±0.20°, 25.46±0.20°, and 25.74±0.20°.

In some embodiments, the X-ray powder diffraction pattern of crystal form C of the monoethanolate of the sodium salt of the compound of formula I, expressed in terms of diffraction angle $2\theta$, is substantially as shown in FIG. 7. In some embodiments, crystal form C of the monoethanolate of the sodium salt of the compound of formula I has a DSC spectrum with a peak at 340.70±5.0 °C. In some embodiments, the DSC spectrum of crystal form C of the monoethanolate of the sodium salt of the compound of formula I is as shown in FIG. 8.

In further another aspect, the present disclosure provides a crystal form of the monohydrate of the sodium salt of the compound of formula I.

In some embodiments, the present disclosure also provides crystal form D of the monohydrate of the sodium salt of the compound of formula I, and the X-ray powder diffraction pattern of crystal form D, expressed in terms of diffraction angle $2\theta$, has diffraction peaks at 8.28±0.20°, 10.08±0.20°, 20.05±0.20°, and 23.47±0.20°.

In some embodiments, the X-ray powder diffraction pattern of crystal form D of the monohydrate of the sodium salt of the compound of formula I, expressed in terms of diffraction angle $2\theta$, has diffraction peaks at 8.28±0.20°, 10.08 ±0.20°, 14.46±0.20°, 16.79±0.20°, 20.05±0.20°, 22.07±0.20°, 22.89±0.20°, and 23.47±0.20°.

In some embodiments, the X-ray powder diffraction pattern of crystal form D of the monohydrate of the sodium salt of the compound of formula I, expressed in terms of diffraction angle $2\theta$, has diffraction peaks at 8.28±0.20°, 10.08 ±0.20°, 14.46±0.20°, 16.67±0.20°, 16.79±0.20°, 17.28±0.20°, 20.05±0.20°, 22.07±0.20°, 22.89±0.20°, 23.47 ±0.20°, and 26.19±0.20°.

In some embodiments, the X-ray powder diffraction pattern of crystal form D of the monohydrate of the sodium salt of the compound of formula I, expressed in terms of diffraction angle $2\theta$, has diffraction peaks at 8.28±0.20°, 10.08 ±0.20°, 12.30±0.20°, 14.01±0.20°, 14.46±0.20°, 16.67±0.20°, 16.79±0.20°, 17.17±0.20°, 17.28±0.20°, 17.45 ±0.20°, 18.15±0.20°, 20.05±0.20°, 22.07±0.20°, 22.42±0.20°, 22.89±0.20°, 23.47±0.20°, 26.19±0.20°, and 26.46 ±0.20°.

In some embodiments, the X-ray powder diffraction pattern of crystal form D of the monohydrate of the sodium salt of the compound of formula I, expressed in terms of diffraction angle $2\theta$, is substantially as shown in FIG. 10. In some embodiments, crystal form D of the monohydrate of the sodium salt of the compound of formula I has a DSC spectrum with a peak at 338.88±5.0 °C. In some embodiments, the DSC spectrum of crystal form D of the monohydrate of the sodium salt of the compound of formula I is as shown in FIG. 11.

In further another aspect, the present disclosure also provides a method for preparing crystal form D of the monohydrate of the sodium salt of the compound of formula I, which comprises: mixing an amorphous form of the sodium salt of the compound of formula I with a mixed solvent of water and a first solvent, stirring, and separating a solid, wherein the first solvent is selected from one or more of toluene, methyl tert-butyl ether, isopropyl ether, or dichloromethane.

In some embodiments, in the method for preparing crystal form D of the monohydrate of the sodium salt of the compound of formula I, the volume (μL) of the mixed solvent of water and the first solvent is 1-30 times or 5-15 times the mass (mg) of the amorphous form of the sodium salt of the compound of formula I.

In some embodiments, the first solvent is selected from toluene. In some embodiments, in the mixed solvent of water and toluene, the content of water is greater than 0 and less than or equal to 0.5 wt%, or is greater than 0 and less than or equal to 0.1 wt%.

In some embodiments, in the method for preparing crystal form D of the monohydrate of the sodium salt of the compound of formula I, the stirring temperature is 0 °C-60 °C or 15 °C-30 °C.

In further another aspect, the present disclosure also provides crystal form E of the sodium salt of the compound of formula I, and the X-ray powder diffraction pattern of crystal form E, expressed in terms of diffraction angle 2$\theta$, has diffraction peaks at 20.40±0.20°, 23.54±0.20°, 24.10±0.20°, and 28.67±0.20°.

In some embodiments, the X-ray powder diffraction pattern of crystal form E of the sodium salt of the compound of formula I, expressed in terms of diffraction angle 2$\theta$, has diffraction peaks at 10.09±0.20°, 17.53±0.20°, 20.40±0.20°, 22.84±0.20°, 23.54±0.20°, 24.10±0.20°, 28.67±0.20°, and 30.58±0.20°.

In some embodiments, the X-ray powder diffraction pattern of crystal form E of the sodium salt of the compound of formula I, expressed in terms of diffraction angle 2$\theta$, has diffraction peaks at 7.95±0.20°, 10.09±0.20°, 11.60±0.20°, 13.93±0.20°, 14.98±0.20°, 16.90±0.20°, 17.53±0.20°, 19.14±0.20°, 19.76±0.20°, 20.40±0.20°, 21.55±0.20°, 22.84 ±0.20°, 23.54±0.20°, 24.10±0.20°, 25.47±0.20°, 25.97±0.20°, 27.27±0.20°, 28.67±0.20°, and 30.58±0.20°.

In some embodiments, the X-ray powder diffraction pattern of crystal form E of the sodium salt of the compound of formula I, expressed in terms of diffraction angle 2$\theta$, is substantially as shown in FIG. 13.

In some embodiments, crystal form E of the sodium salt of the compound of formula I has a DSC spectrum with a peak at 338.17±5.0 °C.

In some embodiments, the DSC spectrum of crystal form E of the sodium salt of the compound of formula I is as shown in FIG. 14.

In further another aspect, the present disclosure also provides an amorphous form of the sodium salt of the compound of formula I.

In some embodiments, the X-ray powder diffraction pattern of the amorphous form of the sodium salt of the compound of formula I, expressed in terms of diffraction angle 2$\theta$, is substantially as shown in FIG. 16.

In some embodiments, the amorphous form of the sodium salt of the compound of formula I has a DSC spectrum with a peak at 337.04 °C±5.0 °C. In some embodiments, the DSC spectrum of the amorphous form of the sodium salt of the compound of formula I is as shown in FIG. 17.

In further another aspect, the present disclosure provides a crystal form of the potassium salt of the compound of formula I.

In further another aspect, the present disclosure provides a crystal form of the monohydrate of the potassium salt of the compound of formula I.

In further another aspect, the present disclosure also provides crystal form G of the monohydrate of the potassium salt of the compound of formula I, and the X-ray powder diffraction pattern of crystal form G, expressed in terms of diffraction angle 2$\theta$, has diffraction peaks at 17.33±0.20°, 20.48±0.20°, 24.07±0.20°, and 25.06±0.20°.

In some embodiments, the X-ray powder diffraction pattern of crystal form G of the monohydrate of the potassium salt of the compound of formula I, expressed in terms of diffraction angle 2$\theta$, has diffraction peaks at 9.06±0.20°, 10.75 ±0.20°, 15.22±0.20°, 17.33±0.20°, 20.48±0.20°, 22.84±0.20°, 23.20±0.20°, 24.07±0.20°, 25.06±0.20°, 28.03 ±0.20°, and 30.18±0.20°.

In some embodiments, the X-ray powder diffraction pattern of crystal form G of the monohydrate of the potassium salt of the compound of formula I, expressed in terms of diffraction angle 2$\theta$, has diffraction peaks at 9.06±0.20°, 10.75 ±0.20°, 12.87±0.20°, 15.22±0.20°, 16.33±0.20°, 17.33±0.20°, 19.09±0.20°, 20.48±0.20°, 21.61±0.20°, 22.84 ±0.20°, 23.20±0.20°, 24.07±0.20°, 25.06±0.20°, 26.01±0.20°, 28.03±0.20°, 28.72±0.20°, 30.18±0.20°, 30.89 ±0.20°, 33.04±0.20°, 33.80±0.20°, and 37.12±0.20°.

In some embodiments, the X-ray powder diffraction pattern of crystal form G of the monohydrate of the potassium salt of the compound of formula I, expressed in terms of diffraction angle 2$\theta$, is substantially as shown in FIG. 19. In some embodiments, crystal form G of the monohydrate of the potassium salt of the compound of formula I has a DSC spectrum with peaks at 110.89 °C±5.0 °C and 336.14±5.0 °C. In some embodiments, the DSC spectrum of crystal form G of the monohydrate of the potassium salt of the compound of formula I is as shown in FIG. 20.

In further another aspect, the present disclosure also provides a method for preparing crystal form G of the monohydrate of the potassium salt of the compound of formula I, which comprises: (1) mixing the compound of formula I with potassium hydroxide in DMF, and (2) adding ethyl acetate, stirring, and separating.

In some embodiments, in the method for preparing crystal form G of the monohydrate of the potassium salt of the compound of formula I, the molar ratio of the compound of formula I to potassium hydroxide is 1:(0.5-1.5), 1:(0.9-1.2), or about 1:1.

In some embodiments, in the method for preparing crystal form G of the monohydrate of the potassium salt of the compound of formula I, the volume (mL) of DMF is 2-20 times or 3-10 times the mass (g) of the compound of formula I in step (1).

In some embodiments, in the method for preparing crystal form G of the monohydrate of the potassium salt of the compound of formula I, the stirring temperature is 20 °C-60 °C or 20 °C-40 °C in step (2).

In further another aspect, the present disclosure also provides an amorphous form of the potassium salt of the compound of formula I, and the X-ray powder diffraction pattern of the amorphous form of the potassium salt of the compound of formula I, expressed in terms of diffraction angle $2\theta$, is substantially as shown in FIG. 22.

In some embodiments, the amorphous form of the potassium salt of the compound of formula I has a DSC spectrum with a peak at 338.12 °C$\pm$5.0 °C. In some embodiments, the DSC spectrum of the amorphous form of the potassium salt of the compound of formula I is as shown in FIG. 23.

The crystallization methods for each crystal form in the present disclosure are conventional, such as solvent evaporation crystallization, cooling crystallization, or crystallization at room temperature.

Further, the preparation methods for each crystal form in the present disclosure further comprise steps such as filtration, washing, or drying.

In another aspect, the present disclosure provides a pharmaceutical composition, which comprises the crystal form of the compound of formula I, or the sodium salt of the compound of formula I, or the crystal form of the solvate of the sodium salt of the compound of formula I, or the potassium salt of the compound of formula I, or the crystal form of the solvate of the potassium salt of the compound of formula I described in the present disclosure, and a pharmaceutically acceptable excipient.

In another aspect, the present disclosure provides a pharmaceutical composition, which comprises the crystal form of the compound of formula I, or the sodium salt of the compound of formula I, or the crystal form of the solvate of the sodium salt of the compound of formula I described in the present disclosure, and a pharmaceutically acceptable excipient.

In another aspect, the present disclosure provides a method for preventing or treating a disease or disorder mediated by DNA polymerase θ in a mammal, which comprises administering to a mammal, preferably a human, in need of the treatment a therapeutically effective amount of the crystal form of the compound of formula I, or the sodium salt of the compound of formula I, or the crystal form of the solvate of the sodium salt of the compound of formula I, or the potassium salt of the compound of formula I, or the crystal form of the solvate of the potassium salt of the compound of formula I, or the pharmaceutical composition thereof described in the present disclosure.

In another aspect, the present disclosure provides a method for preventing or treating a disease or disorder mediated by DNA polymerase θ in a mammal, which comprises administering to a mammal, preferably a human, in need of the treatment a therapeutically effective amount of the crystal form of the compound of formula I, or the sodium salt of the compound of formula I, or the crystal form of the solvate of the sodium salt of the compound of formula I, or the pharmaceutical composition thereof described in the present disclosure.

In another aspect, the present disclosure provides use of the crystal form of the compound of formula I, or the sodium salt of the compound of formula I, or the crystal form of the solvate of the sodium salt of the compound of formula I, or the potassium salt of the compound of formula I, or the crystal form of the solvate of the potassium salt of the compound of formula I, or the pharmaceutical composition thereof described in the present disclosure in preparing a medicament for preventing or treating a disease or disorder mediated by DNA polymerase θ.

In another aspect, the present disclosure provides use of the crystal form of the compound of formula I, or the sodium salt of the compound of formula I, or the crystal form of the solvate of the sodium salt of the compound of formula I, or the pharmaceutical composition thereof described in the present disclosure in preparing a medicament for preventing or treating a disease or disorder mediated by DNA polymerase θ.

In another aspect, the present disclosure provides use of the crystal form of the compound of formula I, or the sodium salt of the compound of formula I, or the crystal form of the solvate of the sodium salt of the compound of formula I, or the potassium salt of the compound of formula I, or the crystal form of the solvate of the potassium salt of the compound of formula I, or the pharmaceutical composition thereof described in the present disclosure in preventing or treating a disease or disorder mediated by DNA polymerase θ.

In another aspect, the present disclosure provides use of the crystal form of the compound of formula I, or the sodium salt of the compound of formula I, or the crystal form of the solvate of the sodium salt of the compound of formula I, or the pharmaceutical composition thereof described in the present disclosure in preventing or treating a disease or disorder mediated by DNA polymerase θ.

In another aspect, the present disclosure provides the crystal form of the compound of formula I, or the sodium salt of the compound of formula I, or the crystal form of the solvate of the sodium salt of the compound of formula I, or the potassium salt of the compound of formula I, or the crystal form of the solvate of the potassium salt of the compound of formula I, or the pharmaceutical composition thereof described in the present disclosure for preventing or treating a disease or disorder mediated by DNA polymerase θ.

In another aspect, the present disclosure provides the crystal form of the compound of formula I, or the sodium

salt of the compound of formula I, or the crystal form of the solvate of the sodium salt of the compound of formula I, or the pharmaceutical composition thereof described in the present disclosure for preventing or treating a disease or disorder mediated by DNA polymerase θ.

In some embodiments, the disease or disorder mediated by DNA polymerase θ is a disease or disorder characterized by overexpression of DNA polymerase θ. In some embodiments, the disease or disorder mediated by DNA polymerase θ is cancer. In some embodiments, the disease or disorder mediated by DNA polymerase θ is a homologous recombination (HR)-deficient cancer. In some embodiments, the cancer is cancer with reduced or absent BRCA gene expression, BRCA gene deficiency, or a reduced BRCA protein function. In some embodiments, the cancer is colorectal cancer. In some embodiments, the cancer is colorectal adenocarcinoma.

The sodium salt of the compound of formula I or the solvate of the sodium salt of the compound of formula I described in the present disclosure, including the crystal form thereof, exhibits good physicochemical stability and offers advantages in terms of physicochemical properties and processability for formulation, making it suitable for preparation into a desired pharmaceutical composition.

## Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered indefinite or unclear, but should be understood according to its common meaning in the field. When referring to a trade name, it is intended to refer to its corresponding commercial product or its active ingredient.

The term "solvate" refers to a complex or aggregate formed by one or more molecules of a solute and one or more molecules of a solvent. Solvates typically have a substantially fixed molar ratio of solute to solvent. The term also includes cage-like compounds, including cage-like compounds having water. Representative solvents include, for example, water, methanol, ethanol, isopropanol, acetic acid, and the like. When the solvent is water, the solvate formed is a hydrate.

The term "monohydrate" means that the molar ratio of water to a compound (or a salt) in the hydrate is about 1:1. The term "about" is used in the present disclosure to mean close to, around, roughly, or approximately. When the term "about" is used in conjunction with a numerical range, the range is modified by extending the upper and lower limits of the stated numerical range. Unless otherwise stated, the term "about" is used herein to modify the upper and lower limits of the stated value by a numerical value that deviates by 10%.

Unless otherwise stated, the term "comprise" and variations thereof, such as "comprises" or "comprising", should be understood in an open and non-exclusive sense, i.e., "including but not limited to".

"Optional embodiment" or "embodiment" mentioned in the present disclosure means that particular reference elements, structures, or features described in connection with the embodiment are included in at least one embodiment. Thus, the appearances of the phrases "optional embodiment" or "embodiment" in various places throughout the present disclosure are not necessarily all referring to the same embodiment. Furthermore, the particular elements, structures, or features may be combined in any suitable manner in one or more embodiments. The room temperature described in the present disclosure refers to 25±5.0 °C.

The range "m-n" described in the present disclosure represents an abbreviated representation of any combination of real numbers between m and n, where m and n are both real numbers. For example, a numerical range of "5-10" indicates that 5, 5.5, 6, 6.5, 7, 7.5, 8, 8.5, 9, 9.5, or 10 has been listed herein; "1-5" indicates that 1, 1.5, 2, 2.5, 3, 3.5, 4, 4.5, or 5 has been listed herein, and "1-5" is only an abbreviated representation of a combination of these numerical values.

The "X-ray powder diffraction pattern" or "XRPD pattern" described in the present disclosure refers to a set of X-ray powder diffraction peaks obtained according to the Bragg's equation $2d \sin\theta = n\lambda$ (where d is the interplanar spacing, $\theta$ is the diffraction angle, $\lambda$ is the wavelength of the incident X-ray, and the diffraction order n is any positive integer (typically n = 1 for first-order diffraction peaks)). When an X-ray is incident on a certain atomic plane with the d-lattice plane spacing of a crystal or part of a crystal sample at a grazing angle $\theta$ (the complementary angle of the incident angle, also known as Bragg angle), the Bragg's equation can be satisfied.

For the same crystal forms of the same compound, the peak positions in the XRPD patterns are similar on the whole, and the relative intensity error may be large. It should also be noted that in the identification of mixtures, some diffraction lines may be absent due to factors such as content reduction, and in this case, it is not necessary to rely on all diffraction peaks observed in a high-purity sample, and even one diffraction peak may be characteristic for a given crystal.

As used in the present disclosure, "$2\theta$ or $2\theta$ angle" refers to a diffraction angle; $\theta$ is the Bragg angle in ° or degrees. To those skilled in the art, due to factors such as crystal defects and measurement errors, the molar ratio of the compound of the present disclosure to the acid/base molecules and that of the compound to the solvent molecules in the solvate often have certain degrees of error. Generally, ±10% falls within a reasonable error range. The error varies to some extent depending on the context in which it is used, and the error variation is no more than ±10%, preferably ±5%.

The term "therapeutically effective amount" refers to an amount of the compound of the present disclosure for (i) treating a specific disease, condition, or disorder; (ii) alleviating, ameliorating, or eliminating one or more symptoms of a specific disease, condition, or disorder; or (iii) delaying the onset of the one or more symptoms of the specific disease, condition, or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the content of the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The pharmaceutical composition of the present disclosure can be prepared by combining the compound or the pharmaceutically acceptable salt thereof or the solvate of the pharmaceutically acceptable salt of the compound of the present disclosure with a suitable pharmaceutically acceptable excipient, and can be formulated, for example, into a solid, semisolid, liquid, or gaseous formulation such as tablets, pills, capsules, powders, granules, ointments, emulsions, suspensions, suppositories, injections, inhalants, gels, microspheres, aerosols, and the like.

Typical routes of administration of the compound or the pharmaceutically acceptable salt thereof or the solvate of the pharmaceutically acceptable salt of the compound, or the pharmaceutical composition comprising the compound or the pharmaceutically acceptable salt thereof or the solvate of the pharmaceutically acceptable salt of the compound of the present disclosure include, but are not limited to, oral, rectal, local, inhalation, parenteral, sublingual, intravaginal, intranasal, intraocular, intraperitoneal, intramuscular, subcutaneous, and intravenous administration.

The pharmaceutical composition of the present disclosure can be manufactured by methods well known in the art, such as conventional methods of mixing, dissolving, granulating, emulsifying, freeze-drying, and the like.

In some embodiments, the pharmaceutical composition is in an oral form. For oral administration, the pharmaceutical composition can be formulated by mixing an active compound with pharmaceutically acceptable excipients well known in the art. These excipients enable the compound or the pharmaceutically acceptable salt thereof or the solvate of the pharmaceutically acceptable salt of the compound of the present disclosure to be formulated into tablets, pills, lozenges, dragees, capsules, liquids, gels, slurries, suspensions, and the like, for oral administration to patients.

A solid oral composition can be prepared by conventional mixing, filling, or tableting. For example, it can be obtained by the following method: mixing the active compound with a solid excipient, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners, flavoring agents, or the like.

The pharmaceutical composition may also be suitable for parenteral administration, such as a sterile solution, suspension, or lyophilized product in a suitable unit dosage form.

The therapeutically effective amount of the crystal form of the compound of formula I, the sodium salt of the compound of formula I, or the solvate of the sodium salt of the compound of formula I contained in the pharmaceutical composition of the present disclosure is selected from 0.001 mg/kg to 1000 mg/kg, in an individual dose or separated doses.

The therapeutically effective amount of the crystal form of the potassium salt of the compound of formula I or the solvate of the potassium salt of the compound of formula I contained in the pharmaceutical composition of the present disclosure is selected from 0.001 mg/kg to 1000 mg/kg, in an individual dose or separated doses.

One skilled in the art recognizes that measured data of XRPD peak positions and/or intensities for a given crystal form of the same compound will vary within a margin of error. The $2\theta$ values in the present disclosure encompass appropriate error ranges, which are generally represented by "±". For example, a $2\theta$ value represented by a specific angle value±0.20° in the present disclosure means that the specific angle value has an error floating range of ±0.20°, that is, 5.92±0.20° $2\theta$ means that $2\theta$ is within the range of 6.12 to 5.72. Depending on the sample preparation technique, the calibration technique applied to the instrument, human operating deviations, and the like, those skilled in the art recognize that an appropriate error range for the XRPD diffraction angles may be ±0.20°, ±0.15°, ±0.10°, ±0.05°, or less, with some variability permitted for the peak intensities. The term "substantially consistent with" or "substantially as shown in ..." when used to describe an XRPD pattern refers to a pattern including diffraction peaks having at least 50%, at least 60%, at least 70%, at least 80%, at least 90%, at least 95%, or at least 99% of the diffraction angles within a standard deviation range of ±0.2° $2\theta$.

As those skilled in the art will recognize, measurement data for a DSC spectrum of a given crystal form of the

same compound will vary within a margin of error. A single peak value (expressed in degrees Celsius) allows for an appropriate error range. Generally, the error range is represented by "±". For the same crystal form of the same compound, the thermal transition temperature and melting point errors in successive analyses are typically within ±5.0 °C. For example, a peak value of "140.96±5.0" means within the range of 145.96 to 135.96. Depending on sample preparation technique, the calibration technique applied to the instrument, human operating variations, and the like, those skilled in the art recognize that appropriate error ranges for single peak values may be ±5.0, ±4.0, ±3.0, ±2.0, or smaller.

The salt forms and/or crystal forms of the present disclosure may also be isotopically labeled. The present disclosure also includes isotopically labeled compounds of the present disclosure that are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$ $^{32}P$, $^{35}S$, $^{18}F$, $^{123}I$, $^{125}I$, and $^{36}Cl$.

Certain isotopically labeled compounds of the present disclosure (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$, and $^{18}F$, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

Furthermore, substitution with heavier isotopes (e.g., deuterium (i.e., $^{2}H$)) may provide certain therapeutic advantages (e.g., increased *in vivo* half-life or reduced dosage) resulting from greater metabolic stability and thus may be preferred in certain circumstances in which deuterium substitution may be partial or complete, wherein partial deuterium substitution refers to substitution of at least one hydrogen with deuterium.

The compound or the pharmaceutically acceptable salt thereof or the solvate of the pharmaceutically acceptable salt of the compound of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compound or the pharmaceutically acceptable salt thereof or the solvate of the pharmaceutically acceptable salt of the compound of the present disclosure, it is sometimes necessary for those skilled in the art to modify or select a synthesis procedure or a reaction process based on the existing embodiments.

Test conditions for the instruments used in the experiments of the present disclosure:

1. X-ray powder diffraction

Instrument model: Bruker D8 Focus
X-ray light source: Cu Kα
Kα1 (Å): 1.54060;
Wavelength λ (Å): 1.54060
Slit (°): 2.5
Scan mode: θ/2θ, scan range: 3-40° (2*θ* angle)
Retention time (s): 0.12
Scanning step length (° 2*θ*): 0.01
Voltage: 40 kV
Current: 40 mA

2. Differential scanning calorimeter

Instrument model: Discovery DSC 2500
Purging gas: nitrogen
Sample tray: aluminum tray, non-sealing gland
Method: linear heating
Ramping rate: 10 °C/min
Temperature range: 30 °C-400 °C

3. Thermogravimetric analyzer

Instrument model: Discovery TA 55
Purging gas: nitrogen
Sample tray: platinum, open
Method: linear heating
Ramping rate: Ramp from a starting temperature of 30 °C to 400 °C at a rate of 10 °C/min
Temperature range: 30 °C-400 °C

4. Dynamic vapor sorption instrument

Instrument model: DVS Intrinsic.

DVS parameters:

Temperature: 25 °C;
Equilibrium: dm/dt = 0.002%/min
RH (%) test gradient: 10%
Range of RH (%) test gradient: 0%-90%-0%.

5. Ion chromatography

Instrument model: Thermo Fisher Integrion, No. IC001
Test method: Conductivity (ELSD)
Separation column: Dionex IonpacTM CS12 Guard 4 × 250 mm
Eluent: 20 mM methanesulfonic acid solution
Flow rate: 1.0 mL/min

The following abbreviations are used in the present disclosure:

| Abbreviation | Chemical name | Abbreviation | Chemical name |
|---|---|---|---|
| HATU | O-(7-Azabenzotriazol-1-yl)-*N,N,N',N'*-tetramethyluronium hexafluorophosphate | DIEA or DIPEA | N,N-Diisopropylethylamine |
| $Pd(dppf)Cl_2$ or $PdCl_2(dppf)$ | 1,1'-Bis(diphenylphosphino)ferrocene dichloropalladium(II) or 1,1-bis(diphenylphosphine)ferrocene palladium chloride | DMF | N,N-Dimethylformamide |
| HOBt | 1-Hydroxybenzotriazole | DMAP | 4-Dimethylaminopyridine |
| DMA | *N,N*-Dimethylacetamide | EDCI | 1-(3-Dimethylaminopropyl)-3-ethylcarbodiimide hydrochloride |
| DMSO | Dimethyl sulfoxide | NMP | N-methyl-2-pyrrolidone |

## BRIEF DESCRIPTION OF THE DRAWINGS

FIG. 1 is the XRPD pattern of crystal form A of the sodium salt of the compound of formula I;
FIG. 2 is the DSC spectrum of crystal form A of the sodium salt of the compound of formula I;
FIG. 3 is the TGA profile of crystal form A of the sodium salt of the compound of formula I;
FIG. 4 is the XRPD pattern of crystal form B of the compound of formula I;
FIG. 5 is the DSC spectrum of crystal form B of the compound of formula I;
FIG. 6 is the TGA profile of crystal form B of the compound of formula I;
FIG. 7 is the XRPD pattern of crystal form C of the monoethanolate of the sodium salt of the compound of formula I;
FIG. 8 is the DSC spectrum of crystal form C of the monoethanolate of the sodium salt of the compound of formula I;
FIG. 9 is the TGA profile of crystal form C of the monoethanolate of the sodium salt of the compound of formula I;
FIG. 10 is the XRPD pattern of crystal form D of the monohydrate of the sodium salt of the compound of formula I;
FIG. 11 is the DSC spectrum of crystal form D of the monohydrate of the sodium salt of the compound of formula I;

FIG. 12 is the TGA profile of crystal form D of the monohydrate of the sodium salt of the compound of formula I;
FIG. 13 is the XRPD pattern of crystal form E of the sodium salt of the compound of formula I;
FIG. 14 is the DSC spectrum of crystal form E of the sodium salt of the compound of formula I;
FIG. 15 is the TGA profile of crystal form E of the sodium salt of the compound of formula I;
FIG. 16 is the XRPD pattern of the amorphous form of the sodium salt of the compound of formula I;
FIG. 17 is the DSC spectrum of the amorphous form of the sodium salt of the compound of formula I;
FIG. 18 is the TGA profile of the amorphous form of the sodium salt of the compound of formula I;
FIG. 19 is the XRPD pattern of crystal form G of the monohydrate of the potassium salt of the compound of formula I;
FIG. 20 is the DSC spectrum of crystal form G of the monohydrate of the potassium salt of the compound of formula I;
FIG. 21 is the TGA profile of crystal form G of the monohydrate of the potassium salt of the compound of formula I;
FIG. 22 is the XRPD pattern of the amorphous form of the potassium salt of the compound of formula I;
FIG. 23 is the DSC spectrum of the amorphous form of the potassium salt of the compound of formula I;
FIG. 24 is the TGA profile of the amorphous form of the potassium salt of the compound of formula I.

## EXAMPLES

The present disclosure is described in detail below by way of examples. However, this is by no means disadvantageously limiting the scope of the present disclosure. Although the present disclosure has been described in detail herein and specific embodiments thereof have also been disclosed, it will be apparent to those skilled in the art that various changes and modifications can be made to the specific embodiments of the present disclosure without departing from the spirit and scope of the present disclosure. All reagents used in the present disclosure are commercially available and can be used without further purification.

Unless otherwise stated, the ratios expressed for mixed solvents are volume mixing ratios.

Compounds are named either manually or by ChemDraw® software, and suppliers' catalog names are given for commercially available compounds.

The structures of the compounds are determined by nuclear magnetic resonance (NMR) and/or mass spectrometry (MS). NMR shifts are given in $10^{-6}$ (ppm). The solvents for NMR determination are deuterated dimethyl sulfoxide, deuterated chloroform, deuterated methanol, and the like, and the internal standard is tetramethylsilane (TMS); "$IC_{50}$" refers to the half inhibitory concentration, which is the concentration at which half of the maximal inhibitory effect is achieved.

In the following purification using high-performance liquid chromatography, "%" for the amount of the acid or base used in the mobile phase A means a volume fraction unless otherwise specified. For example, "water (0.05% formic acid)" means that the volume of formic acid is 0.05% of the total volume of formic acid and water. B% represents the ratio of the volume of mobile phase B to the total volume of mobile phase A and mobile phase B during gradient elution, and "B%: 50%-70%" represents that the ratio of the volume of mobile phase B to the total volume of mobile phase A and mobile phase B changes from 50% to 70% during gradient elution.

### Example 1. Synthesis of 4-(5-Chloro-2-methoxyphenyl)-N-[6-(4-cyanophenyl)thiazolo[4,5-b]pyrazin-2-yl]-6-methylnicotinamide (Compound of Formula I)

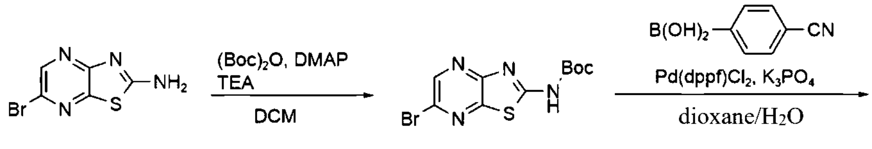


1 2

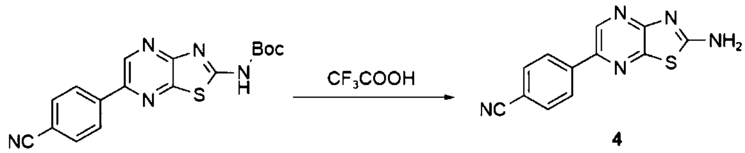


3 4

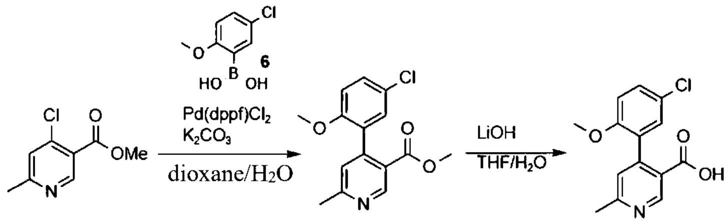


5 7 8

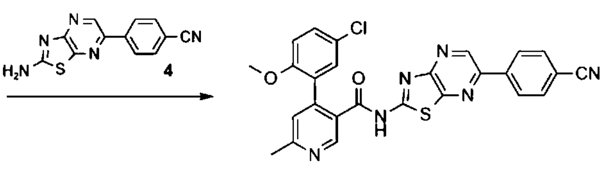


(I)

**Step 1: Synthesis of tert-butyl (6-bromothiazolo[4,5-b]pyrazin-2-yl)carbamate (intermediate 2)**

**Starting material 1** (790 mg), triethylamine (691.90 mg), and DMAP (41.77 mg) were dissolved in dichloromethane (10 mL), $(Boc)_2O$ (820.78 mg) was added dropwise to the reaction mixture, and the mixture was stirred at room temperature for 16 h. Then, the reaction mixture was washed with saturated brine, the organic phase was dried over anhydrous sodium sulfate, filtered, and the filtrate was concentrated under reduced pressure to give the title compound (1 g). MS m/z (ESI): 331.2/333.2 $[M+H]^+$.

**Step 2: Synthesis of tert-butyl (6-(4-cyanophenyl)thiazolo[4,5-b]pyrazin-2-yl)carbamate (intermediate 3)**

**Intermediate 2** (1 g), 4-cyanobenzeneboronic acid (887.34 mg), Pd(dppf)$Cl_2$ (220.93 mg), and potassium phosphate (1.28 g) were added to dioxane (10 mL) and water (2 mL), and the mixture was stirred at 80 °C for 4 h. The reaction mixture was poured into water, and the mixture was extracted with ethyl acetate (10 mL × 3). The organic phase was dried over anhydrous sodium sulfate and filtered, and the filtrate was concentrated to dryness under reduced pressure. The residue was slurried with ethyl acetate and filtered, and the filter cake was dried to give the title compound (0.5 g). MS m/z (ESI): 354.0 $[M+H]^+$.

**Step 3: Synthesis of 4-(2-aminothiazolo[4,5-b]pyrazin-6-yl)benzonitrile (intermediate 4)**

**Intermediate 3** (0.48 g) was dissolved in trifluoroacetic acid (2 mL), and the mixture was stirred at room temperature for 1 h. Ethyl acetate was added to the reaction mixture, and the mixture was filtered. The filtrate was concentrated to dryness under reduced pressure to give the title compound (230 mg). MS m/z (ESI): 254.0 $[M+H]^+$.

**Step 4: Synthesis of methyl 4-(5-chloro-2-methoxyphenyl)-6-methylpyridine-3-carboxylate (intermediate 7)**

Under a nitrogen atmosphere, **intermediate 6** (1 g) was dissolved in dioxane (20 mL) and water (5 mL), and **intermediate 5** (995.75 mg), Pd(dppf)$Cl_2$ (349.65 mg), and potassium carbonate (1.48 g) were added to the reaction mixture. Then, the reaction mixture was stirred at 90 °C for 2 h under a nitrogen atmosphere. After the reaction was completed, water (50 mL) was added to the reaction mixture. The mixture was extracted with ethyl acetate (50 mL × 3 times). The organic phases were combined and dried over anhydrous sodium sulfate. After filtration, the organic phase was concentrated under reduced pressure to remove the solvent, and the residue was purified by silica gel column chromatography (ISCO®; 20 g SepaFlash® Silica Flash chromatographic column, gradient: 0-50% ethyl acetate/petroleum ether, flow rate: 20 mL/min) to give the title compound (870 mg). MS m/z (ESI): 292.1 $[M+H]^+$.

**Step 5: Synthesis of 4-(5-chloro-2-methoxyphenyl)-6-methylpyridine-3-carboxylic acid (intermediate 8)**

**Intermediate 7** (870 mg) was added to tetrahydrofuran (8 mL) and water (4 mL), lithium hydroxide (157.13 mg) was added to the reaction mixture, and then the reaction mixture was stirred at 25 °C for 16 h. After the reaction was completed, the pH of the reaction mixture was adjusted to 3, and the solvent was removed by concentration under reduced pressure. The residue was washed with a mixed solvent of dichloromethane/methanol (10/1, 20 mL) and filtered, and the filtrate was concentrated to dryness under reduced pressure to give the title compound (1.2 g). MS m/z (ESI): 277.9 $[M+H]^+$.

**Step 6: Synthesis of 4-(5-chloro-2-methoxyphenyl)-*N*-16-(4-cyanophenyl)thiazolo[4,5-*b*]pyrazin-2-yl]-6-methylnicotinamide (compound of formula I)**

Under a nitrogen atmosphere, **intermediate 8** (300 mg) was added to N,N-dimethylformamide (5 mL), and **intermediate 4** (273.62 mg), HATU (410.76 mg), and N,N-diisopropylethylamine (279.24 mg) were added to the reaction mixture. Then, the reaction mixture was stirred at 25 °C for 2 h under a nitrogen atmosphere. After the reaction was completed, the reaction mixture was purified by preparative high-performance liquid chromatography (chromatographic column: Boston Prime C18 150 × 30 mm × 5 μm; mobile phase: [A: water (0.225% formic acid), B: acetonitrile]; B%: 48%-68%, 11 min) to give the title compound (51.96 mg). MS m/z (ESI): 513.1 $[M+H]^+$. **$^1$H NMR (400MHz, DMSO-*d*$_6$)** δ 13.40 (brs, 1H), 9.27 (s, 1H), 8.84 (s, 1H), 8.36 (d, *J* = 8.4 Hz, 2H), 8.00 (d, *J* = 8.3 Hz, 2H), 7.50-7.42 (m, 2H), 7.36 (s, 1H), 7.03-6.99 (m, 1H), 3.52 (s, 3H), 2.59 (s, 3H)

**Example 2. Preparation of Crystal Form A of Sodium Salt of Compound of Formula I**

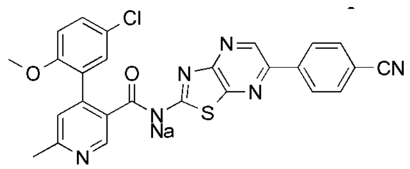

9.5 g of the compound of formula I was suspended in 190 mL of absolute ethanol, and a solution of sodium hydroxide (742 mg) in absolute ethanol (190 mL) was added. After the addition, the mixture was stirred at 25 °C for 3 h. The reaction mixture was filtered, the filter cake was washed with absolute ethanol (40 mL) and collected. The filter cake was dried under vacuum at 50 °C for 12 h to give crystal form A of the sodium salt of the compound of formula I. $^1$H NMR (400 MHz, DMSO-d6) δ 9.02 (s, 1H), 8.96 (s, 1H), 8.28 (d, J = 8.5 Hz, 2H), 7.92 (d, J = 8.5 Hz, 2H), 7.37 (m, 1H), 7.24 (d, J = 2.7 Hz, 1H), 7.13 (s, 1H), 6.99 (d, J = 8.9 Hz, 1H), 3.52 (s, 3H), 2.53 (s, 3H).

Crystal form A of the sodium salt of the compound of formula I was prepared by the method of Example 2. The crystal sample has an XRPD pattern shown in FIG. 1, a DSC spectrum shown in FIG. 2, and a TGA profile shown in FIG. 3. The sodium ion content determined by ion chromatography is 4.2%, which corresponds to a molar ratio of 1:1 between the compound of formula I to sodium (the theoretical sodium ion content is 4.3%). DSC exhibits a peak at about 342.84 °C, and the XRPD diffraction peak parameters are shown in the table below.

| Peak No. | 2$\theta$ value [° or degree] | d[Å] | Net intensity (counts) | Relative intensity (%) |
|---|---|---|---|---|
| 1 | 6.129 | 14.40949 | 577.857 | 30.3 |

(continued)

| Peak No. | 2$\theta$ value [° or degree] | d[Å] | Net intensity (counts) | Relative intensity (%) |
|---|---|---|---|---|
| 2 | 6.386 | 13.82918 | 1022.08 | 53.6 |
| 3 | 6.872 | 12.85307 | 238.285 | 12.5 |
| 4 | 7.836 | 11.27313 | 1907.25 | 100.0 |
| 5 | 10.795 | 8.18940 | 70.9205 | 3.7 |
| 6 | 13.613 | 6.49959 | 1172.10 | 61.5 |
| 7 | 14.099 | 6.27645 | 410.284 | 21.5 |
| 8 | 15.945 | 5.55378 | 605.973 | 31.8 |
| 9 | 16.439 | 5.38812 | 236.792 | 12.4 |
| 10 | 17.164 | 5.16205 | 570.856 | 29.9 |
| 11 | 17.737 | 4.99653 | 266.749 | 14.0 |
| 12 | 19.085 | 4.64649 | 58.7828 | 3.1 |
| 13 | 20.895 | 4.24802 | 278.914 | 14.6 |
| 14 | 21.812 | 4.07131 | 226.315 | 11.9 |
| 15 | 22.010 | 4.03512 | 176.028 | 9.2 |
| 16 | 22.726 | 3.90975 | 119.869 | 6.3 |
| 17 | 22.925 | 3.87622 | 173.851 | 9.1 |
| 18 | 25.458 | 3.49602 | 1045.68 | 54.8 |
| 19 | 30.793 | 2.90137 | 225.996 | 11.8 |

**Example 3. Crystal Form B of Compound of Formula I**

Intermediate 4 (10.00 g, 1.00 eq.), intermediate 8 (16.45 g, 1.50 eq.), HOBt (8.54 g, 1.60 eq.), DMF (85 mL, 8.5 V), and DIPEA (10.21 g, 2.00 eq.) were added to a reaction kettle, and then EDCI (11.35 g, 1.50 eq.) was added. The mixture was stirred at a controlled temperature of 25±5 °C, reacted for 15 h, and then sampled for in-process control. After the reaction was completed, the mixture was cooled to 0-10 °C, stirred for another 1-2 h, and filtered, and the filter cake was rinsed with DMF (1 V) and absolute ethanol (2 V), respectively, to give a wet filter cake. The wet filter cake was dried under vacuum to constant weight in a vacuum drying box at 55±5 °C to give 14.28 g of crystal form B of the compound of formula I. $^1$H NMR (400 MHz, DMSO-d6) $\delta$ 13.43 (s, 1H), 9.36 (s, 1H), 8.80 (s, 1H), 8.38 (d, J = 8.5 Hz, 2H), 8.02 (d, J = 8.5 Hz, 2H), 7.50 (d, J = 2.6 Hz, 1H), 7.49 - 7.44 (m, 1H), 7.42 (s, 1H), 7.02 (d, J = 8.8 Hz, 1H), 3.52 (s, 3H), 2.61 (s, 3H).

Crystal form B of the compound of formula I was prepared by the method of Example 3. The crystal sample has an XRPD pattern shown in FIG. 4, a DSC spectrum shown in FIG. 5, and a TGA profile shown in FIG. 6. DSC exhibits a peak at about 343.98 °C, and the XRPD diffraction peak parameters are shown in the table below.

| Peak No. | 2$\theta$ value [° or degree] | d [Å] | Net intensity (counts) | Relative intensity (%) |
|---|---|---|---|---|
| 1 | 7.983 | 11.06624 | 246 | 26.8 |
| 2 | 12.100 | 7.30871 | 366 | 39.9 |
| 3 | 14.586 | 6.06802 | 270 | 29.4 |
| 4 | 15.972 | 5.54449 | 183 | 19.9 |
| 5 | 17.314 | 5.11762 | 134 | 14.6 |
| 6 | 17.794 | 4.98064 | 918 | 100.0 |
| 7 | 18.228 | 4.86299 | 120 | 13.1 |
| 8 | 20.134 | 4.40680 | 547 | 59.6 |
| 9 | 22.511 | 3.94659 | 279 | 30.4 |

(continued)

| Peak No. | 2$\theta$ value [° or degree] | d [Å] | Net intensity (counts) | Relative intensity (%) |
|---|---|---|---|---|
| 10 | 23.313 | 3.81252 | 224 | 24.4 |
| 11 | 23.612 | 3.76494 | 298 | 32.5 |
| 12 | 23.980 | 3.70793 | 206 | 22.4 |
| 13 | 24.562 | 3.62142 | 209 | 22.8 |
| 14 | 25.470 | 3.49435 | 690 | 75.2 |
| 15 | 26.743 | 3.33083 | 506 | 55.1 |
| 16 | 27.659 | 3.22251 | 395 | 43.0 |

**Example 4. Crystal Form C of Monoethanolate of Sodium Salt of Compound of Formula** I

Preparation method I:

3 g of the compound of formula I and 15 mL of DMSO were added to a reaction flask, and the mixture was stirred at room temperature. A solution of sodium hydroxide (257 mg) in ethanol (9 mL) was added, and the reaction mixture was dissolved completely and heated to 45 °C in a water bath. 30 mL of ethanol was added, and the mixture was slowly cooled to room temperature and filtered by suction. The filter cake was rinsed with absolute ethanol and dried under vacuum at 40 °C to give crystal form C of the ethanolate of the sodium salt of the compound of formula I (which is the crystal form of the monoethanolate of the sodium salt of the compound of formula I). $^1$H NMR (400 MHz, DMSO-d6) $\delta$ 9.02 (s, 1H), 8.95 (s, 1H), 8.27 (d, J = 8.5 Hz, 2H), 7.91 (d, J = 8.5 Hz, 2H), 7.48 - 7.30 (m, 1H), 7.23 (d, J = 2.7 Hz, 1H), 7.12 (s, 1H), 6.98 (d, J = 8.9 Hz, 1H), 3.51 (s, 3H), 2.53 (s, 3H).

Crystal form C of the monoethanolate of the sodium salt of the compound of formula I was prepared by the method of Example 4. The crystal sample has an XRPD pattern shown in FIG. 7, a DSC spectrum shown in FIG. 8, and a TGA profile shown in FIG. 9. DSC exhibits a peak at about 340.70 °C, and the XRPD diffraction peak positions are shown in the table below.

| Peak No. | 2$\theta$ value [° or degree] | d [Å] | Net intensity (counts) | Relative intensity (%) |
|---|---|---|---|---|
| 1 | 12.374 | 7.14725 | 92.7 | 31.75 |
| 2 | 12.697 | 6.96637 | 78.0 | 26.71 |
| 3 | 15.466 | 5.72472 | 82.8 | 28.36 |
| 4 | 17.139 | 5.16944 | 209 | 71.58 |
| 5 | 17.955 | 4.93648 | 103 | 35.27 |
| 6 | 19.756 | 4.49018 | 79.6 | 27.26 |
| 7 | 20.065 | 4.42172 | 146 | 50.00 |
| 8 | 20.303 | 4.37044 | 137 | 46.92 |
| 9 | 21.397 | 4.14940 | 144 | 49.32 |
| 10 | 21.846 | 4.06513 | 239 | 81.85 |
| 11 | 22.285 | 3.98601 | 59.0 | 20.21 |
| 12 | 23.388 | 3.80054 | 145 | 49.66 |
| 13 | 24.507 | 3.62947 | 186 | 63.70 |
| 14 | 24.990 | 3.56031 | 196 | 67.12 |
| 15 | 25.463 | 3.49527 | 292 | 100.00 |
| 16 | 25.742 | 3.45800 | 237 | 81.16 |

Preparation method II:

3 g of the compound of formula I and 15 mL of NMP or DMF or DMA were added to a reaction flask, and the mixture was stirred at room temperature. A solution of sodium hydroxide (257 mg) in ethanol (9 mL) was added, and the reaction mixture was dissolved completely and heated to 45 °C in a water bath. 30 mL of ethanol was added, and the mixture was slowly cooled to 25 °C and filtered by suction. The filter cake was rinsed with absolute ethanol and dried under vacuum at 40 °C to give crystal form C of the monoethanolate of the sodium salt of the compound of formula I.

Preparation method III:

0.5 g of the compound of formula I and 1.8 mL of DMSO were added to a reaction flask, and the mixture was stirred at room temperature. 0.35 g of a 20% aqueous sodium ethoxide solution was added, and the reaction mixture was dissolved completely and heated to 45 °C in a water bath. 30 mL of ethanol was added, and the mixture was slowly cooled to 25 °C and filtered by suction. The filter cake was rinsed with absolute ethanol and dried under vacuum at 40 °C to give crystal form C of the monoethanolate of the sodium salt of the compound of formula I.

**Example 5. Crystal Form D of Monohydrate of Sodium Salt of Compound of Formula I**

10 mg of the amorphous form of the sodium salt of the compound of formula I was added to a reaction flask, and 100 μL of toluene with a water content of 0.08 wt% (the solvent may also be methyl tert-butyl ether, isopropyl ether, or dichloromethane with a water content of about 0.1 wt%) was added. The mixture was stirred at 25 °C overnight and filtered by suction, and the filter cake was dried at room temperature under vacuum to give crystal form D of the monohydrate of the sodium salt of the compound of formula I. $^1H$ NMR (400 MHz, DMSO-d6) δ 9.02 (s, 1H), 8.95 (s, 1H), 8.28 (d, J = 8.6 Hz, 2H), 7.91 (d, J = 8.6 Hz, 2H), 7.36 (m, 1H), 7.23 (d, J = 2.7 Hz, 1H), 7.12 (s, 1H), 6.98 (d, J = 8.8 Hz, 1H), 3.52 (s, 3H), 2.53 (s, 3H).

Crystal form D was prepared by the method of Example 5. The crystal sample has an XRPD pattern shown in FIG. 10, a DSC spectrum shown in FIG. 11, and a TGA profile shown in FIG. 12. DSC exhibits a peak at about 338.88 °C, and the XRPD diffraction peak parameters of the XRPD diffraction peaks it comprises are shown in the table below. The sodium ion content determined by ion chromatography is 3.7%, which corresponds to a molar ratio of 1:1 between the compound of formula I to sodium (the theoretical sodium ion content is 4.2%). Crystal form D exhibits excellent physical stability, remaining stable in properties with no form conversion under conditions such as light, high temperature, and high humidity.

| Peak No. | 2$\theta$ value [° or degree] | d [Å] | Net intensity (counts) | Relative intensity (%) |
|---|---|---|---|---|
| 1 | 8.283 | 10.66593 | 1394.22 | 38.8 |
| 2 | 10.075 | 8.77283 | 2432.51 | 67.7 |
| 3 | 12.298 | 7.19111 | 404.668 | 11.3 |
| 4 | 14.014 | 6.31427 | 600.584 | 16.7 |
| 5 | 14.461 | 6.12040 | 997.779 | 27.8 |
| 6 | 16.674 | 5.31252 | 791.206 | 22.0 |
| 7 | 16.787 | 5.27705 | 943.613 | 26.3 |
| 8 | 17.171 | 5.15985 | 657.951 | 18.3 |
| 9 | 17.276 | 5.12890 | 835.586 | 23.3 |
| 10 | 17.453 | 5.07732 | 360.524 | 10.0 |
| 11 | 18.153 | 4.88304 | 383.900 | 10.7 |
| 12 | 20.051 | 4.42491 | 3425.52 | 95.3 |
| 13 | 22.073 | 4.02383 | 1368.06 | 38.1 |
| 14 | 22.418 | 3.96261 | 493.834 | 13.7 |
| 15 | 22.885 | 3.88281 | 2389.84 | 66.5 |
| 16 | 23.473 | 3.78688 | 3592.87 | 100.0 |
| 17 | 26.194 | 3.39937 | 719.353 | 20.0 |
| 18 | 26.461 | 3.36566 | 608.562 | 16.9 |

**Example 6. Crystal Form E of Sodium Salt of Compound of Formula I**

10 mg of the amorphous form of the sodium salt of the compound of formula I was added to a reaction flask, and 100 μL of ethyl acetate was added. The mixture was stirred at room temperature overnight and filtered by suction, and the filter cake was dried at room temperature under vacuum to give crystal form E of the sodium salt of the compound of formula I. $^{1}H$ NMR (400 MHz, DMSO-d6) δ 9.01 (s, 1H), 8.98 (s, 1H), 8.28 (d, J = 8.5 Hz, 2H), 7.92 (d, J = 8.4 Hz, 2H), 7.45 - 7.33 (m, 1H), 7.25 (d, J = 2.7 Hz, 1H), 7.14 (s, 1H), 6.99 (d, J = 8.8 Hz, 1H), 3.52 (s, 3H), 2.54 (s, 3H).

Crystal form E of the sodium salt of the compound of formula I was prepared by the method of Example 6. The crystal sample has an XRPD pattern shown in FIG. 13, a DSC spectrum shown in FIG. 14, and a TGA profile shown in FIG. 15. DSC exhibits a peak at about 338.17 °C, and the XRPD diffraction peak parameters are shown in the table below. Crystal form E exhibits relatively poor physical stability under high humidity (92.5% RH) and accelerated (40 °C, 75% RH) conditions.

| Peak No. | 2$\theta$ value [° or degree] | d [Å] | Net intensity (counts) | Relative intensity (%) |
|---|---|---|---|---|
| 1 | 7.947 | 11.11611 | 132 | 21.53 |
| 2 | 10.085 | 8.76378 | 138 | 22.51 |
| 3 | 11.598 | 7.62373 | 71.9 | 11.73 |
| 4 | 13.933 | 6.35107 | 81.4 | 13.28 |
| 5 | 14.979 | 5.90962 | 43.2 | 7.05 |
| 6 | 16.896 | 5.24322 | 110 | 17.94 |
| 7 | 17.525 | 5.05639 | 204 | 33.28 |
| 8 | 19.135 | 4.63446 | 42.2 | 6.88 |
| 9 | 19.760 | 4.48924 | 51.8 | 8.45 |
| 10 | 20.400 | 4.34997 | 613 | 100.00 |
| 11 | 21.549 | 4.12045 | 85.8 | 14.00 |
| 12 | 22.844 | 3.88974 | 146 | 23.82 |
| 13 | 23.540 | 3.77627 | 320 | 52.20 |
| 14 | 24.095 | 3.69051 | 603 | 98.37 |
| 15 | 25.470 | 3.49429 | 115 | 18.76 |
| 16 | 25.966 | 3.42877 | 101 | 16.48 |
| 17 | 27.273 | 3.26730 | 85.6 | 13.96 |
| 18 | 28.668 | 3.11135 | 316 | 51.55 |
| 19 | 30.576 | 2.92140 | 135 | 22.02 |

**Example 7. Amorphous Form of Sodium Salt of Compound of Formula I**

1 g of crystal form C of the monoethanolate of the sodium salt of the compound of formula I (or any one of crystal form A of the sodium salt of the compound of formula I, crystal form D of the monohydrate of the sodium salt of the compound of formula I, and crystal form E of the sodium salt of the compound of formula I) was added to a single-necked flask. 60 mL of hexafluoroisopropanol was added, and the mixture was stirred until complete dissolution and then rotary-evaporated to dryness to give the amorphous form of the sodium salt of the compound of formula I.

The amorphous sample of the sodium salt of the compound of formula I was prepared by the method of Example 7. The crystal sample has an XRPD pattern shown in FIG. 16, a DSC spectrum shown in FIG. 17, and a TGA profile shown in FIG. 18. DSC exhibits a peak at about 337.04 °C.

**Example 8. Crystal Form G of Monohydrate of Potassium Salt of Compound of Formula I**

100 mg of the compound of formula I, 0.5 mL of N,N'-dimethylformamide, and 13 mg of potassium hydroxide were added to a centrifuge tube and stirred at room temperature, and the reaction mixture was dissolved completely. 2 mL of

ethyl acetate was added, and the mixture was stirred at room temperature. After a large amount of solid was precipitated, the reaction mixture was filtered by suction, and the filter cake was dried under vacuum at 45 °C to give crystal form G of the monohydrate of the potassium salt of the compound of formula I.

Crystal form G of the monohydrate of the potassium salt of the compound of formula I was prepared by the method of Example 8. The crystal sample has an XRPD pattern shown in FIG. 19, a DSC spectrum shown in FIG. 20, and a TGA profile shown in FIG. 21. DSC exhibits peaks at about 110.89 °C and 336.14 °C, and the XRPD diffraction peak parameters are shown in the table below.

| Peak No. | 2$\theta$ value [° or degree] | d [Å] | Net intensity (counts) | Relative intensity (%) |
|---|---|---|---|---|
| 1 | 9.056 | 9.75694 | 51.7 | 22.00 |
| 2 | 10.746 | 8.22652 | 59.3 | 25.23 |
| 3 | 12.872 | 6.87181 | 21.2 | 9.02 |
| 4 | 15.222 | 5.81587 | 89.9 | 38.26 |
| 5 | 16.334 | 5.42224 | 27.9 | 11.87 |
| 6 | 17.326 | 5.11400 | 94.2 | 40.09 |
| 7 | 19.092 | 4.64489 | 31.0 | 13.19 |
| 8 | 20.479 | 4.33322 | 235 | 100.00 |
| 9 | 21.614 | 4.10823 | 30.4 | 12.94 |
| 10 | 22.843 | 3.88991 | 66.5 | 28.30 |
| 11 | 23.203 | 3.83032 | 65.0 | 27.66 |
| 12 | 24.074 | 3.69374 | 158 | 67.23 |
| 13 | 25.060 | 3.55056 | 209 | 88.94 |
| 14 | 26.009 | 3.42317 | 37.0 | 15.74 |
| 15 | 28.033 | 3.18045 | 101 | 42.98 |
| 16 | 28.723 | 3.10557 | 40.9 | 17.40 |
| 17 | 30.178 | 2.95909 | 75.0 | 31.91 |
| 18 | 30.887 | 2.89277 | 36.5 | 15.53 |
| 19 | 33.040 | 2.70900 | 49.7 | 21.15 |
| 20 | 33.795 | 2.65017 | 33.7 | 14.34 |
| 21 | 37.124 | 2.41980 | 37.3 | 15.87 |

**Example 9. Amorphous Form of Potassium Salt of Compound of Formula I**

100 mg of the compound of formula I, 0.5 mL of N,N'-dimethylformamide, and 13 mg of potassium hydroxide were added to a centrifuge tube and stirred at room temperature, and the reaction mixture was dissolved completely. 2 mL of ethanol was added, and the mixture was stirred at room temperature. After a large amount of solid was precipitated, the reaction mixture was filtered by suction, and the filter cake was dried under vacuum at 45 °C to give the amorphous form of the potassium salt of the compound of formula I.

The amorphous sample of the potassium salt of the compound of formula I was prepared by the method of Example 9. The crystal sample has an XRPD pattern shown in FIG. 22, a DSC spectrum shown in FIG. 23, and a TGA profile shown in FIG. 24. DSC exhibits a peak at about 338.12 °C.

**Experimental Example 10. Influencing Factor and Accelerated Experiments**

Each test sample was placed under high temperature (60 °C), high humidity (92.5%), light (7000 lux), or accelerated (40 °C, 75% RH) conditions using a 2 mL screw-thread sample vial in an open state; each sample was placed with packaging (2 layers of medicinal low-density polyethylene bags + 1 layer of polyester/aluminum/polyethylene medicinal composite film bag) under light (7000 lux) or accelerated (40 °C, 75% RH) conditions to investigate the stability

of the sample. The results are shown in the table below.

Results of influencing factor experiment

| Sample | Storage condition | Investigation time point | Purity (%) | Total impurities (%) |
|---|---|---|---|---|
| Crystal form A | 0 h | 0d | 99.21 | 0.79 |
| | High temperature (60 °C) | 7d | 99.23 | 0.77 |
| | | 14d | 99.17 | 0.83 |
| | | 32d | 99.15 | 0.85 |
| | High humidity (92.5% RH) | 7d | 99.26 | 0.74 |
| | | 14d | 99.20 | 0.80 |
| | | 32d | 99.21 | 0.79 |
| | Light (7000 lux, open) | 7d | 99.11 | 0.89 |
| | | 14d | 98.75 | 1.25 |
| | | 32d | 98.24 | 1.76 |
| | Light (7000 lux, packaged) | 10d | 99.19 | 0.81 |
| | | 32d | 99.22 | 0.78 |
| Crystal form B | 0 h | 0d | 98.89 | 1.11 |
| | High temperature (60 °C) | 7d | 98.98 | 1.02 |
| | | 14d | 98.93 | 1.07 |
| | | 32d | 98.84 | 1.16 |
| | High humidity (92.5% RH) | 7d | 98.60 | 1.40 |
| | | 14d | 98.89 | 1.11 |
| | | 32d | 98.83 | 1.17 |
| | Light (7000 lux, open) | 7d | 98.84 | 1.16 |
| | | 14d | 98.85 | 1.15 |
| | | 32d | 98.73 | 1.27 |
| | Light (7000 lux, packaged) | 10d | 98.90 | 1.10 |
| | | 32d | 98.90 | 1.10 |
| Crystal form C | 0h | 0d | 99.2 | 0.80 |
| | High temperature (60 °C) | 7d | 99.26 | 0.74 |
| | | 14d | 99.22 | 0.78 |
| | High humidity (92.5% RH) | 7d | 99.21 | 0.79 |
| | | 14d | 99.20 | 0.80 |
| | Light (7000 lux, open) | 7d | 98.29 | 1.71 |
| | | 14d | 97.16 | 2.84 |

(continued)

| Sample | Storage condition | Investigation time point | Purity (%) | Total impurities (%) |
|---|---|---|---|---|
| Crystal form D | 0h | 0d | 99.48 | 0.52 |
| | High temperature (60 °C) | 7d | 99.49 | 0.51 |
| | | 14d | 99.46 | 0.54 |
| | High humidity (92.5% RH) | 7d | 99.51 | 0.49 |
| | | 14d | 99.46 | 0.54 |
| | Light (7000 lux, open) | 7d | 99.39 | 0.61 |
| | | 14d | 99.20 | 0.80 |
| Crystal form E | 0h | 0d | 99.39 | 0.52 |
| | High temperature (60 °C) | 11d | 99.25 | 0.75 |
| | High humidity (92.5% RH) | 11d | 99.48 | 0.52 |
| | Light (7000 lux, open) | 7d | 99.24 | 0.76 |
| Amorphous form of sodium salt of compound of formula I | 0h | 0d | 99.31 | 0.52 |
| | High temperature (60 °C) | 11d | 99.23 | 0.77 |
| | High humidity (92.5% RH) | 11d | 98.89 | 1.11 |
| | Light (7000 lux, open) | 7d | 97.57 | 2.43 |

Results of accelerated experiment

| Sample | Storage condition | Investigation time point | Purity (%) | Total impurities (%) |
|---|---|---|---|---|
| Crystal form A | 0 h | 0d | 99.21 | 0.79 |
| | Accelerated (40 °C), open | 7d | 99.21 | 0.79 |
| | | 14d | 99.17 | 0.83 |
| | | 32d | 99.18 | 0.82 |
| | Accelerated (40 °C), packaged | 14d | 99.19 | 0.81 |
| | | 32d | 99.19 | 0.81 |
| Crystal form B | 0 h | 0d | 98.89 | 1.11 |
| | Accelerated (40 °C), open | 7d | 98.84 | 1.16 |
| | | 14d | 98.87 | 1.13 |
| | | 32d | 98.88 | 1.12 |
| | Accelerated (40 °C), packaged | 14d | 98.87 | 1.13 |
| | | 32d | 98.82 | 1.18 |
| Crystal form C | 0h | 0d | 99.2 | 0.8 |
| | Accelerated (40 °C), open | 7d | 99.20 | 0.80 |
| | | 14d | 99.21 | 0.79 |
| Crystal form D | 0h | 0d | 99.48 | 0.52 |
| | Accelerated (40 °C), open | 7d | 99.48 | 0.52 |
| | | 14d | 99.45 | 0.55 |

(continued)

| Sample | Storage condition | Investigation time point | Purity (%) | Total impurities (%) |
|---|---|---|---|---|
| Crystal form E | 0h | 0d | 99.39 | 0.52 |
| | Accelerated (40 °C), open | 7d | 99.49 | 0.51 |
| Amorphous form of sodium salt of compound of formula I | 0h | 0d | 99.31 | 0.52 |
| | Accelerated (40 °C), open | 7d | 99.33 | 0.67 |

**Experimental Example 11. Solubility Experiment**

The thermodynamic solubility of the compounds of the present disclosure was determined using the following test method.

I. Preparation of dissolution medium

Purified water: self-made in the laboratory

II. Experimental procedures

The thermodynamic solubility of the compounds of the present disclosure in water was investigated. Water (1000 μL) was measured and placed into a 1 mL transparent glass tube with a stopper. An appropriate amount of each sample was weighed and added to saturate the solution, and the solution was shaken to disperse the sample. Each sample was placed on a roller mixer and shaken in water for 24 h. Then, an appropriate amount of the sample was taken, filtered through a microporous filter membrane, and subjected to content determination using high-performance liquid chromatography with an external standard method. The thermodynamic solubility of each compound in water (μg/mL) was calculated. The solubility results are detailed in the table below.

| Vehicle system | Dynamic solubility in water (μg/mL) | | | |
|---|---|---|---|---|
| | 1h | 2h | 4h | 24h |
| Crystal form A | 2081.71 | 1590.35 | 1441.04 | 27.01 |
| Crystal form B | <0.5 | <0.5 | <0.5 | 0.77 |
| Crystal form C | 857.52 | / | 1337.74 | 108.15 |
| Crystal form E | <0.5 | 1.03 | 2.26 | 2.72 |
| Amorphous form of sodium salt of compound of formula I | 1.57 | 4.37 | 44.68 | 0.72 |

**Experimental Example 12. Hygroscopicity Experiment**

(1) Experimental instrument: dynamic vapor sorption DVS Intrinsic.
(2) Experimental conditions: Each test sample was taken and placed in a DVS sample pan for testing.
(3) DVS parameters:

Temperature: 25 °C;
Equilibrium: dm/dt = 0.002%/min
RH (%) test gradient: 10%
Range of RH (%) test gradient: 0%-90%-0%.

Experimental results:

Under the condition of 80.0% RH, the weight gain due to moisture absorption for crystal form A is 1.6%.

Under the condition of 80.0% RH, the weight gain due to moisture absorption for crystal form B is 1.5%.
Under the condition of 80.0% RH, the weight gain due to moisture absorption for crystal form C is -4.3%, with solvent desorption being observed during the cycle.
Under the condition of 80.0% RH, the weight gain due to moisture absorption for crystal form E is 2.5%.

**Test Examples of Biological Activity and Related Properties**

The compounds in the following test examples were prepared according to the methods in the above examples disclosed in the present disclosure.

**Test Example 1. POLQ Enzyme Activity Inhibition Experiment**

Brief introduction **to experimental principle:** After the N-terminal active peptide segment of POLQ with ATPase activity (M1-N899) is co-incubated with the compound, this peptide segment reacts with substrate dT50 under the action of ATP to generate ADP. The generated ADP then participates in the subsequent NADH oxidation-coupled enzymatic reaction, catalyzing the conversion of NADH to $NAD^+$. The reduction in the OD value of NADH at 340 nm was measured using the Envision microplate reader from Perkin Elmer to reflect enzyme activity.
**Experimental instruments:** Labcyte Echo 650 pipetting system; Perkin Elmer Envision microplate reader; Eppendorf 5810R centrifuge; Boxun BSD-YX3400 thermostatic shaker.

**Experimental materials:**

| Reagent | Brand |
|---|---|
| POLQ (N) | Synthesized by Pharmaron |
| ATP | Sigma |
| dT50 | Synthesized by GenScript |
| NADH | Roche |
| PEP | Sigma |
| Lactate dehydrogenase | Sigma |
| Pyruvate kinase | Sigma |
| 384-well plate | Greiner Bio-One |

**Experimental methods:** The POLQ enzyme was diluted to 100 nM with a reaction buffer (20 mM Tris HCl (pH 7.80), 80 mM KCl, 10 mM $MgCl_2$, 1 mM DTT, 0.01% w/v bovine serum albumin, 0.01% v/v Tween-20, 5% v/v glycerol). The test compound was diluted to different concentrations with dimethyl sulfoxide (DMSO) using the Echo 650 pipetting system and transferred to a 384-well plate, 20 μL/well of 100 nM POLQ was added, and the mixture was incubated at room temperature for 15 min. A reaction mixture solution was prepared with the concentrations of each component in the reaction mixture solution as follows: 100 μM ATP, 300 nM dT50, 300 μM NADH, 6 mM PEP, 10 U/mL lactate dehydrogenase, and 20 U/mL pyruvate kinase. 20 μL/well of the reaction mixture solution was added to start the enzyme reaction. In the reaction system, the compound was serially diluted 3-fold from the final concentration of 10 μM, covering a concentration range from 10 μM to 0.0005 μM, and the final concentration of DMSO in the system was 0.2% v/v. After the 384-well plate was reacted at room temperature for 20 min, the OD value at 340 nm was read using an Envision microplate reader.

**Data analysis:**

Inhibition rates were calculated and fitted using XLfit software to give the $IC_{50}$ of the compound.
A blank group and a DMSO group were set in the experiment. The reaction system of the blank group was 0.2% v/v DMSO and the reaction mixture solution, and the inhibition rate was considered to be 100% under this condition.
The reaction system of the DMSO group was 0.2% v/v DMSO, POLQ (N) (100 nM), and the reaction mixture solution, and the inhibition rate was considered to be 0 under this condition.

$$\text{Inhibition rate} = (100 - 100 \times (OD_{max} - OD_{compound})/(OD_{max} - OD_{min}))\%$$

wherein $OD_{max}$ refers to the OD value of the well containing the reactant mixture solution and 0.2% v/v DMSO, and $OD_{compound}$ refers to the OD value of the well containing the compound, the enzyme, and the reactant mixture solution. $OD_{min}$ refers to the OD value of the well containing the enzyme, the reactant mixture solution, and 0.2% v/v DMSO.

The biological activity of the compounds of the present disclosure was determined by the above test, and the measured $IC_{50}$ values are shown in Table 1 below.

Table 1. $IC_{50}$ of example compounds on inhibition of POLO enzyme activity

| Example compound number | $IC_{50}$ (nM) |
|---|---|
| Compound of formula I | ++++ |

In the above table, the symbols used to indicate the inhibitory activity represent the following meanings:

"++++" indicates that the $IC_{50}$ range of the test compound for enzyme inhibitory activity is: $IC_{50}$ < 100 nM.
"+++" indicates that the $IC_{50}$ range of the test compound for enzyme inhibitory activity is: 100 ≤ $IC_{50}$ < 500 nM.
"++" indicates that the $IC_{50}$ range of the test compound for enzyme inhibitory activity is: 500 ≤ $IC_{50}$ < 1000 nM.

**Test Example 2. Inhibitory Experiment of Compounds on Tumor Cell Proliferation**

**Brief introduction to experimental principle:** After the compound was co-incubated with tumor cells for 7 days, the ATP in viable cells was quantified using the CTG kit from Promega to reflect the effect of the compound on tumor cell proliferation.

**Experimental instruments:** Perkin Elmer Envision microplate reader; Eppendorf 5810R centrifuge; Countstar automatic cell counter.

**Experimental materials:**

| Reagent | Brand | Catalog No. |
|---|---|---|
| DLD-1 parent cells | Horizon | HD PAR-008 |
| DLD-1 BRCA2 (-/-) cell line | Horizon | HD 105-007 |
| RPMI 1640 medium | Gibco | A10491-01 |
| FBS | Gibco | 10099-141C |
| 0.25% Trypsin-EDTA | Gibco | 25200-172 |
| PBS | HyClone | SH30256.01 |
| 96-well plate | Corning | 3610 |
| CelltiterGlo assay kit (CTG) | Promega | G7573 |

**Experimental methods:** DLD-1 parent cells or DLD-1 BRCA2 (-/-) cells were diluted with an RPMI 1640 medium containing 10% FBS and then added to a 96-well plate (90 μL/well), with the number of cells being 600/well or 1200/well, respectively, and incubated overnight in an incubator at 37 °C with 5% $CO_2$. The test compound was diluted to different concentrations with dimethyl sulfoxide (DMSO) and then added to a 96-well plate, such that, in the reaction system, the compound was serially diluted 4-fold from the final concentration of 25 μM. The concentration range of the compound was from 25 μM to 0.0004 μM, and the final concentration of DMSO was 0.25% v/v. After incubation for 7 days, 50 μL/well of CTG was added, and the mixture was incubated at room temperature for 10 min. The luminescence (Lum) signal value was read using an Envision microplate reader, and the inhibition rate and half inhibitory concentration ($IC_{50}$) were calculated.

**Data analysis:**

Inhibition rates were calculated and fitted using XLfit software to give the $IC_{50}$ of the compound.

A Blank well and a DMSO well were set in the experiment. The blank well contained 100 μL of an RPMI Medium 1640 medium containing 10% FBS, and the inhibition rate of the compound on tumor cell growth was considered to be 100% under this condition. For the DMSO well, 0.25% v/v DMSO was added to the cell-containing well, and the inhibition rate of the compound on tumor cell growth was considered to be 0 under this condition.

$$\text{Inhibition rate} = 100 \times (Lum_{max} - Lum_{compound})/(Lum_{max} - Lum_{min})\%$$

wherein $Lum_{max}$ refers to the luminescence signal value of the well containing cells and 0.25% v/v DMSO, and $Lum_{compound}$ refers to the luminescence signal value of the well containing the compound and cells. $Lum_{min}$ refers to the luminescence signal value of the well containing the medium and 0.25% v/v DMSO.

The growth inhibition of the compounds of the present disclosure on tumor cells was determined by the above test, and an $IC_{50}$ value was determined.

Table 2. $IC_{50}$ of example compounds on inhibition of tumor cell growth

| Example compound number | DLD-1 BRCA2 (-/-) $IC_{50}$ (nM) | DLD-1 parent cells $IC_{50}$ (nM) |
|---|---|---|
| Compound of formula I | ++++ | - |

In the above table, the symbols used to indicate the inhibitory activity represent the following meanings:

"++++" indicates that the $IC_{50}$ range of the test compound for cell inhibitory activity is: $IC_{50}$ < 200 nM.
"+++" indicates that the $IC_{50}$ range of the test compound for cell inhibitory activity is: 200 nM ≤ $IC_{50}$ < 500 nM.
"++" indicates that the $IC_{50}$ range of the test compound for cell inhibitory activity is: 500 nM ≤ $IC_{50}$ < 1000 nM.
"+" indicates that the $IC_{50}$ range of the test compound for cell inhibitory activity is: 1000 nM ≤ $IC_{50}$ < 10000 nM.
"-" indicates that the $IC_{50}$ range of the test compound for cell inhibitory activity is: $IC_{50}$ ≥ 10000 nM.

As tested, the compounds of formula I of the present disclosure have good inhibitory effect on tumor cells with BRCA2 mutation, and have good selectivity.

**Test Example 3. Experiment on Inhibition of Compounds on Cellular MMEJ Pathway**

**Brief introduction to experimental principle:** POLQ is a key protein in the repair process of cellular MMEJ. The NanoLuciferase MMEJ repair reporter system was transferred into HEK293T cells. When the MMEJ repair pathway was normally performed in the cells, the NanoLuciferase reporter protein was correctly expressed, and the cell luminescence signal could be detected. The decrease in cell luminescence was measured using a BMG multifunctional microplate reader from BMG LABTECH to reflect the inhibition of the compound on the cellular MMEJ pathway.

**Experimental instruments:** ESCD Incucyte viable cell imaging system, Labcyte Echo 655 pipetting system, BMG LABTECH BMG multifunctional microplate reader, and Invitrogen Neon transfection system.

**Experimental materials:**

| Reagent | Brand | Catalog No. |
|---|---|---|
| MMEJ luciferase substrate | Synthesized by ICE Bioscience | / |
| HEK293T cell | ATCC | CRL-3216 |
| Nano-Glo Luciferase Assay kit | Promega | N1130 |
| DMEM | Gibco | 11995-065 |
| DMSO | Solarbio | D8371 |
| DPBS | Gibco | 14190250 |
| TrypL Express | Gibco | 25200-072 |

(continued)

| Reagent | Brand | Catalog No. |
|---|---|---|
| 384-well plate | Corning | 3764 |
| Neon™ Transfection System 100 μL Kit | Invitrogen | MPK10096 |

**Experimental methods:** The test compound was diluted to different concentrations with dimethyl sulfoxide (DMSO) using an Echo 655 pipetting system and transferred to a 384-well plate. In the reaction system, the compound was serially diluted 3-fold from the final concentration of 10 μM. The final concentration of DMSO was 0.1%. HEK293T cells were collected, an MMEJ luciferase substrate was transferred to the cells using a Neon transfection system, and the transfected HEK293T cells at 4000 cells/well were diluted with a DMEM medium containing 10% FBS and added to a 384-well plate (25 μL/well). After the compound and the cells were incubated in an incubator at 37 °C with 5% $CO_2$ for 24 h, 40 μL/well of a NanoGlo substrate buffer was added to measure the growth inhibition of the compound on tumor cells, and the inhibition rate and half inhibitory concentration ($IC_{50}$) were calculated.

**Data analysis:**

The compound inhibition rate was calculated and fitted using XLfit software to give the $IC_{50}$ of the compounds. A Blank well and a DMSO well were set in the experiment. 10 μM positive compound ART558 (doi: 10.1038/s41467-021-23463-8) was added to the blank well, and the compound inhibition rate was considered to be 100% under this condition. 0.1% DMSO was added to the DMSO well, and the compound inhibition rate was considered to be 0 under this condition.

$$\text{Compound inhibition rate (\%)} = (100 \times (\text{DMSO well - test compound well})/(\text{DMSO well - blank well}))\%$$

As tested, the compounds of formula I of the present disclosure have strong inhibitory activity on the POLQ-mediated MMEJ pathway in cells, and the compounds are expected to effectively inhibit the target POLQ and the related pathway MMEJ in tumors, thereby exerting corresponding pharmacological effects.

**Test Example 4. Determination of Metabolic Stability of Compounds in Hepatocytes**

The metabolic stability of the compounds of the present disclosure in hepatocytes was determined by the following test method.

I. Experimental materials and instruments

1. Caucasian human hepatocytes (Biopredic BQHPCH10), cynomolgus monkey hepatocytes (RILD HP-SXH-02M), Beagle dog hepatocytes (BioIVT M00205), SD rat hepatocytes (BioIVT M00005), and CD-1 mouse hepatocytes (BioIVT M00505)
2. AOPI stain (Nexcelom 200710-01-01)
3. Dexamethasone (NIFDC 100129-201506)
4. DPBS (10×) (Gibco by Life Technologies 2060570)
5. Fetal bovine serum (FBS) (Corning 35081001)
6. GlutaMAXTM-1 (100×) (Gibco by Life Technologies 2186980)
7. HEPES (Sigma RNBJ1276)
8. Human recombinant insulin (Gibco by Life Technologies 2090407)
9. Isotonic Percoll (GE Healthcare 10288259)
10. Verapamil (Sigma MKBV4993V)
11. Williams' Medium E (Sigma RNBJ3314)
12. AB Sciex API4000 liquid chromatography-mass spectrometry system

II. Experimental procedures

1. The hepatocyte thawing medium was prepared according to the information in the table below. 49.5 mL of Williams'

Medium E and 0.5 mL of GlutaMAXTM-1 (100×) were mixed as an incubation medium. The hepatocyte thawing medium and incubation medium were pre-heated in a water bath at 37 °C for at least 15 min prior to use. A tube of hepatocytes stored at an ultra-low temperature was taken, and the hepatocytes were ensured to be at a frozen state at a low temperature prior to thawing. The hepatocytes were quickly placed in a water bath at 37 °C and gently shaken until all ice crystals were completely dispersed. The hepatocytes were sprayed with 70% ethanol and transferred into a biological safety cabinet. The contents of the tube of hepatocytes were poured into a centrifuge tube containing 50 mL of the thawing medium and centrifuged at 100 g for 10 min. After centrifugation, the thawing medium was aspirated, and a sufficient amount of the incubation medium was added to give a cell suspension with a cell density of about 1.5 × $10^6$ cells/mL. Cellometer Vision was used to count hepatocytes and determine viable cell density; the viability of hepatocytes must be greater than 75%. The hepatocyte suspension was diluted by using the incubation medium to a viable cell density of 0.5 × $10^6$ viable cells/mL.

| Reagent | Initial concentration | Final concentration | Volume of reagent |
|---|---|---|---|
| Williams' Medium E | - | - | 31.2 mL |
| Isotonic Percoll | - | 30% | 13.5 mL |
| DPBS (10×) | - | - | 1.5 mL |
| GlutaMAXTM-1(100×) | 200 mM/100× | 2 mM | 500 μL |
| HEPES | 1 M | 15 mM | 750 μL |
| FBS | - | 5% | 2.5 mL |
| Human recombinant insulin | 4 mg/mL | 4 μg/mL | 50 μL |
| Dexamethasone | 10 mM | 1 μM | 5 μL |

2. 247.5 μL of the viable cell suspension or medium was transferred to a 96-well deep-well plate, and the deep-well plate was vortexed and placed in an incubator and preheated for 10 min. Incubation of all the samples was performed in parallel duplicate. 2.5 μL of the 100 μM test compound or control drug verapamil was added to each well to start the reaction, and the deep-well plate was placed back in the vortex incubator. 25 μL of the incubation sample was taken at 0 min, 15 min, 30 min, 60 min, 90 min, and 120 min, separately, and 125 μL of acetonitrile containing an internal standard was added to terminate the reaction. The mixture was vortexed for 10 min and centrifuged at 3220 g at 4 °C for 30 min. After the centrifugation was completed, 100 μL of the supernatant was transferred to an injection plate, and 150 μL of pure water was added. The mixture was mixed well for LC-MS/MS analysis.

All data were calculated by Microsoft Excel software. The peak area was detected through an extracted ion chromatogram. The *in vitro* half-life ($t_{1/2}$) of the compound was determined by performing linear fitting of the natural logarithm of elimination percent of the compound and time.

*In vitro* half-life ($t_{1/2}$) was calculated by the slope:

$$\textit{in vitro}\ t_{1/2} = 0.693/k$$

The *in vitro* intrinsic clearance (unit: μL/min/mg protein) was calculated using the following formula:

$$\textit{in vitro}\ CL_{int} = k \times \text{volume of incubation } (\mu L)/\text{amount of proteins (mg)}$$

$CL_{int}$ is the intrinsic clearance rate; *k* is an elimination rate constant; volume of incubation is the incubation volume (μL); amount of proteins is the amount of the protein (mg).

As tested, the compounds of formula I of the present disclosure are metabolically stable in hepatocytes of various species, are expected to be relatively stable *in vivo* through liver metabolism, and are relatively less affected by the first-pass effect of the liver.

The embodiments of the present disclosure have been described above. However, the present disclosure is not limited to the embodiments described above. Any modification, equivalent replacement, improvement, etc., made within the spirit and principle of the present disclosure should be included in the protection scope of the present disclosure.

## Claims

**1.** A sodium salt of a compound of formula I having the following structure,

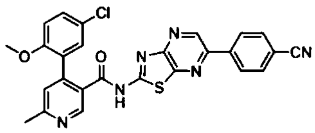

I.

2. The sodium salt according to claim 1, wherein a molar ratio of the compound of formula I to sodium ions is about 1:1; or, a molar ratio of the compound of formula I to sodium ions is 1:1.

3. The sodium salt according to any one of claims 1-2, wherein the sodium salt is an amorphous form or a crystal form; or, the sodium salt is a crystal form; or the sodium salt is an amorphous form.

4. The sodium salt according to claim 3, wherein the sodium salt is crystal form A of the sodium salt of the compound of formula I, and an X-ray powder diffraction pattern of crystal form A, expressed in terms of diffraction angle $2\theta$, has diffraction peaks at 6.39±0.20°, 7.84±0.20°, 13.61±0.20°, and 25.46±0.20°; or,

an X-ray powder diffraction pattern of crystal form A, expressed in terms of diffraction angle $2\theta$, has diffraction peaks at 6.39±0.20°, 7.84±0.20°, 13.61±0.20°, 15.95±0.20°, 17.16±0.20°, 20.90±0.20°, 21.81±0.20°, 25.46 ±0.20°, and 30.79±0.20°; or,
an X-ray powder diffraction pattern of crystal form A, expressed in terms of diffraction angle $2\theta$, has diffraction peaks at 6.13±0.20°, 6.39±0.20°, 6.87±0.20°, 7.84±0.20°, 10.80±0.20°, 13.61±0.20°, 14.10±0.20°, 15.95 ±0.20°, 16.44±0.20°, 17.16±0.20°, 17.74±0.20°, 19.09±0.20°, 20.90±0.20°, 21.81±0.20°, 22.01±0.20°, 22.73±0.20°, 22.93±0.20°, 25.46±0.20°, and 30.79±0.20°; or
an X-ray powder diffraction pattern of crystal form A, expressed in terms of diffraction angle $2\theta$, is substantially as shown in FIG. 1.

5. The sodium salt according to claim 4, wherein crystal form A has a DSC spectrum with a peak at 342.84±5.0 °C.

6. The sodium salt according to claim 3, wherein the sodium salt is crystal form E of the sodium salt of the compound of formula I, and an X-ray powder diffraction pattern of crystal form E, expressed in terms of diffraction angle $2\theta$, has diffraction peaks at 20.40±0.20°, 23.54±0.20°, 24.10±0.20°, and 28.67±0.20°; or

an X-ray powder diffraction pattern of crystal form E, expressed in terms of diffraction angle $2\theta$, has diffraction peaks at 10.09±0.20°, 17.53±0.20°, 20.40±0.20°, 22.84±0.20°, 23.54±0.20°, 24.10±0.20°, 28.67±0.20°, and 30.58±0.20°; or
an X-ray powder diffraction pattern of crystal form E, expressed in terms of diffraction angle $2\theta$, has diffraction peaks at 7.95±0.20°, 10.09±0.20°, 11.60±0.20°, 13.93±0.20°, 14.98±0.20°, 16.90±0.20°, 17.53±0.20°, 19.14±0.20°, 19.76±0.20°, 20.40±0.20°, 21.55±0.20°, 22.84±0.20°, 23.54±0.20°, 24.10±0.20°, 25.47 ±0.20°, 25.97±0.20°, 27.27±0.20°, 28.67±0.20°, and 30.58±0.20°; or
an X-ray powder diffraction pattern of crystal form E, expressed in terms of diffraction angle $2\theta$, is substantially as shown in FIG. 13.

7. The sodium salt according to claim 6, wherein crystal form E has a DSC spectrum with a peak at 338.17±5.0 °C.

8. The sodium salt according to claim 3, wherein the sodium salt is an amorphous form of the sodium salt of the compound of formula I; an X-ray powder diffraction pattern of the amorphous form, expressed in terms of diffraction angle $2\theta$, is substantially as shown in FIG. 16.

9. The sodium salt according to claim 8, wherein the amorphous form has a DSC spectrum with a peak at 337.04 °C±5.0 °C.

10. The sodium salt according to any one of claims 1-2, wherein the sodium salt is a solvate of the sodium salt of the compound of formula I; or the sodium salt is a hydrate or an ethanolate of the sodium salt of the compound of formula I.

11. The sodium salt according to claim 10, wherein a molar ratio of the sodium salt of the compound of formula I to the solvent in the solvate is about 1:1; or, a molar ratio of the sodium salt of the compound of formula I to the solvent in the solvate is 1:1.

**12.** The sodium salt according to any one of claims 10-11, wherein the solvate is a monohydrate of the sodium salt of the compound of formula I; or, the solvate is crystal form D of the monohydrate of the sodium salt of the compound of formula I, wherein an X-ray powder diffraction pattern of crystal form D, expressed in terms of diffraction angle $2\theta$, has diffraction peaks at 8.28±0.20°, 10.08±0.20°, 20.05±0.20°, and 23.47±0.20°; or, an X-ray powder diffraction pattern of crystal form D, expressed in terms of diffraction angle $2\theta$, has diffraction peaks at 8.28±0.20°, 10.08±0.20°, 14.46 ±0.20°, 16.79±0.20°, 20.05±0.20°, 22.07±0.20°, 22.89±0.20°, and 23.47±0.20°; or, an X-ray powder diffraction pattern of crystal form D, expressed in terms of diffraction angle $2\theta$, has diffraction peaks at 8.28±0.20°, 10.08±0.20°, 14.46±0.20°, 16.67±0.20°, 16.79±0.20°, 17.28±0.20°, 20.05±0.20°, 22.07±0.20°, 22.89±0.20°, 23.47±0.20°, and 26.19±0.20°; or, an X-ray powder diffraction pattern of crystal form D, expressed in terms of diffraction angle $2\theta$, has diffraction peaks at 8.28±0.20°, 10.08±0.20°, 12.30±0.20°, 14.01±0.20°, 14.46±0.20°, 16.67±0.20°, 16.79 ±0.20°, 17.17±0.20°, 17.28±0.20°, 17.45±0.20°, 18.15±0.20°, 20.05±0.20°, 22.07±0.20°, 22.42±0.20°, 22.89 ±0.20°, 23.47±0.20°, 26.19±0.20°, and 26.46±0.20°; or, an X-ray powder diffraction pattern of crystal form D, expressed in terms of diffraction angle $2\theta$, is substantially as shown in FIG. 10.

**13.** The sodium salt according to claim 12, wherein crystal form D has a DSC spectrum with a peak at 338.88±5.0 °C.

**14.** The sodium salt according to any one of claims 10-11, wherein the solvate is a monoethanolate of the sodium salt of the compound of formula I; or, the solvate is crystal form C of the monoethanolate of the sodium salt of the compound of formula I, wherein an X-ray powder diffraction pattern of crystal form C, expressed in terms of diffraction angle $2\theta$, has diffraction peaks at 17.14±0.20°, 21.85±0.20°, 25.46±0.20°, and 25.74±0.20°; or, an X-ray powder diffraction pattern of crystal form C, expressed in terms of diffraction angle $2\theta$, has diffraction peaks at 12.37±0.20°, 17.14 ±0.20°, 20.07±0.20°, 21.40±0.20°, 21.85±0.20°, 23.39±0.20°, 24.51±0.20°, 24.99±0.20°, 25.46±0.20°, and 25.74±0.20°; or, an X-ray powder diffraction pattern of crystal form C, expressed in terms of diffraction angle $2\theta$, has diffraction peaks at 12.37±0.20°, 12.70±0.20°, 15.47±0.20°, 17.14±0.20°, 17.96±0.20°, 19.76±0.20°, 20.07 ±0.20°, 20.30±0.20°, 21.40±0.20°, 21.85±0.20°, 22.29±0.20°, 23.39±0.20°, 24.51±0.20°, 24.99±0.20°, 25.46 ±0.20°, and 25.74±0.20°; or, an X-ray powder diffraction pattern of crystal form C, expressed in terms of diffraction angle $2\theta$, is substantially as shown in FIG. 7.

**15.** The sodium salt according to claim 14, wherein crystal form C has a DSC spectrum with a peak at 340.70±5.0 °C.

**16.** A preparation method for crystal form A of the sodium salt of the compound of formula I according to claim 4, comprising: (1) mixing the compound of formula I with absolute ethanol, and (2) adding a mixed solution of sodium hydroxide and absolute ethanol, stirring, and separating.

**17.** The method according to claim 16, wherein a molar ratio of the compound of formula I to sodium hydroxide is 1:(0.5-1.5), 1:(0.9-1.2), or about 1:1; and/or, a volume (mL) of absolute ethanol is 20-45 times or 35-45 times a mass (g) of the compound of formula I in step (1); and/or, the stirring is performed at a temperature of 20-60 °C or 20-30 °C in step (2).

**18.** A preparation method for crystal form D of the monohydrate of the sodium salt of the compound of formula I according to claim 12, comprising: mixing an amorphous form of the sodium salt of the compound of formula I with a mixed solvent of water and toluene, stirring, and separating a solid.

**19.** The method according to claim 18, wherein a volume (μL) of the mixed solvent of water and toluene is 1-30 times or 5-15 times a mass (mg) of the amorphous form of the sodium salt of the compound of formula I, and/or, the stirring is performed at a temperature of 0-60 °C or 15-30 °C.

**20.** A crystal of a compound of formula I:

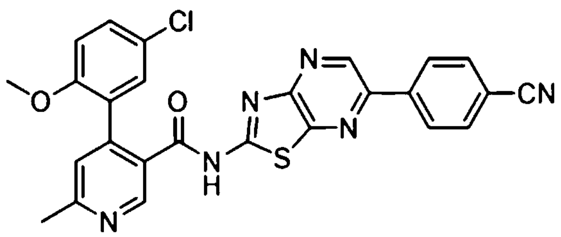

I.

**21.** The crystal of the compound of formula I according to claim 20, wherein the crystal is crystal form B of the compound of formula I, wherein an X-ray powder diffraction pattern of crystal form B, expressed in terms of diffraction angle 2$\theta$, has diffraction peaks at 12.10±0.20°, 17.79±0.20°, 20.13±0.20°, and 25.47±0.20°; or, an X-ray powder diffraction pattern of crystal form B, expressed in terms of diffraction angle 2$\theta$, has diffraction peaks at 7.98±0.20°, 12.10±0.20°, 14.59±0.20°, 17.80±0.20°, 20.13±0.20°, 22.51±0.20°, 23.61±0.20°, 25.47±0.20°, 26.74±0.20°, and 27.66 ±0.20°; or, an X-ray powder diffraction pattern of crystal form B, expressed in terms of diffraction angle 2$\theta$, has diffraction peaks at 7.98±0.20°, 12.10±0.20°, 14.59±0.20°, 15.97±0.20°, 17.31±0.20°, 17.79±0.20°, 18.23 ±0.20°, 20.13±0.20°, 22.51±0.20°, 23.31±0.20°, 23.61±0.20°, 23.98±0.20°, 24.56±0.20°, 25.47±0.20°, 26.74 ±0.20°, and 27.66±0.20°; or, an X-ray powder diffraction pattern of crystal form B, expressed in terms of diffraction angle 2$\theta$, is substantially as shown in FIG. 4.

**22.** The crystal of the compound of formula I according to claim 21, wherein crystal form B has a DSC spectrum with a peak at 343.98±5.0 °C.

**23.** A potassium salt of a compound of formula I having the following structure,

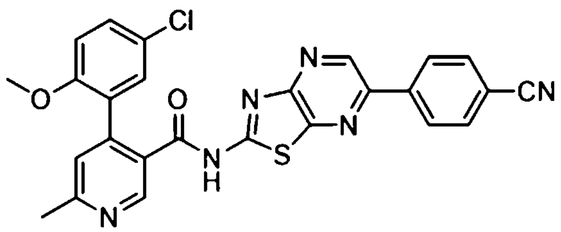

I.

**24.** The potassium salt according to claim 23, wherein a molar ratio of the compound of formula I to potassium ions is about 1:1; or, a molar ratio of the compound of formula I to potassium ions is 1:1.

**25.** The potassium salt according to any one of claims 23-24, wherein the potassium salt is an amorphous form or a crystal form; or, the potassium salt is a crystal form.

**26.** The potassium salt according to any one of claims 23-25, wherein the potassium salt is a solvate of the potassium salt of the compound of formula I; or the potassium salt is a hydrate or an ethanolate of the potassium salt of the compound of formula I.

**27.** The potassium salt according to claim 26, wherein in the solvate of the potassium salt of the compound of formula I, a molar ratio of the potassium salt of the compound of formula I to the solvent is about 1:1; or, a molar ratio of the potassium salt of the compound of formula I to the solvent is 1:1.

**28.** The potassium salt according to claim 27, wherein the solvate is a monohydrate of the potassium salt of the compound of formula I; or, the solvate is crystal form G of the monohydrate of the potassium salt of the compound of formula I, wherein an X-ray powder diffraction pattern of crystal form G, expressed in terms of diffraction angle 2$\theta$, has diffraction peaks at 17.33±0.20°, 20.48±0.20°, 24.07±0.20°, and 25.06±0.20°; or, an X-ray powder diffraction pattern of crystal form G, expressed in terms of diffraction angle 2$\theta$, has diffraction peaks at 9.06±0.20°, 10.75±0.20°, 15.22±0.20°, 17.33±0.20°, 20.48±0.20°, 22.84±0.20°, 23.20±0.20°, 24.07±0.20°, 25.06±0.20°, 28.03±0.20°, and 30.18 ±0.20°; or, an X-ray powder diffraction pattern of crystal form G, expressed in terms of diffraction angle 2$\theta$, has diffraction peaks at 9.06±0.20°, 10.75±0.20°, 12.87±0.20°, 15.22±0.20°, 16.33±0.20°, 17.33±0.20°, 19.09 ±0.20°, 20.48±0.20°, 21.61±0.20°, 22.84±0.20°, 23.20±0.20°, 24.07±0.20°, 25.06±0.20°, 26.01±0.20°, 28.03 ±0.20°, 28.72±0.20°, 30.18±0.20°, 30.89±0.20°, 33.04±0.20°, 33.80±0.20°, and 37.12±0.20°; or, an X-ray powder diffraction pattern of crystal form G, expressed in terms of diffraction angle 2$\theta$, is substantially as shown in FIG. 19.

**29.** The potassium salt according to claim 28, wherein crystal form G has a DSC spectrum with peaks at 110.89 °C±5.0 °C and 336.14±5.0 °C.

**30.** The potassium salt according to claim 25, wherein the potassium salt is an amorphous form of the potassium salt of the compound of formula I; an X-ray powder diffraction pattern of the amorphous form, expressed in terms of diffraction

angle 2θ, is substantially as shown in FIG. 22.

**31.** The potassium salt according to claim 30, wherein the amorphous form has a DSC spectrum with a peak at 338.12 °C ±5.0 °C.

**32.** A pharmaceutical composition, comprising the sodium salt according to any one of claims 1-15, the crystal of the compound of formula I according to any one of claims 20-22, or the potassium salt according to any one of claims 23-31, and a pharmaceutically acceptable excipient.

**33.** A method for preventing or treating a disease mediated by DNA polymerase θ in a mammal, comprising administering to a mammal, preferably a human, in need a therapeutically effective amount of the sodium salt according to any one of claims 1-15, the crystal of the compound of formula I according to any one of claims 20-22, or the potassium salt according to any one of claims 23-31, or the pharmaceutical composition according to claim 32.

**34.** Use of the sodium salt according to any one of claims 1-15, the crystal of the compound of formula I according to any one of claims 20-22, or the potassium salt according to any one of claims 23-31, or the pharmaceutical composition according to claim 32 in preparing a medicament for preventing or treating a disease mediated by DNA polymerase θ.

**35.** Use of the sodium salt according to any one of claims 1-15, the crystal of the compound of formula I according to any one of claims 20-22, or the potassium salt according to any one of claims 23-31, or the pharmaceutical composition according to claim 32 in preventing or treating a disease or disorder mediated by DNA polymerase θ.

**36.** The sodium salt according to any one of claims 1-15, the crystal of the compound of formula I according to any one of claims 20-22, or the potassium salt according to any one of claims 23-31, or the pharmaceutical composition according to claim 32 for use in preventing or treating a disease or disorder mediated by DNA polymerase θ.

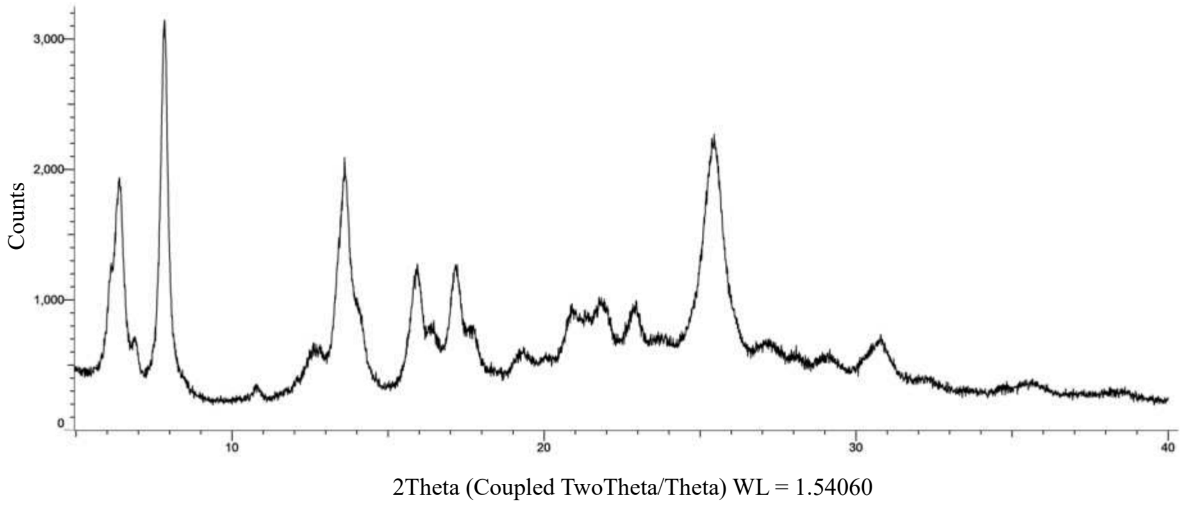
Counts
3,000
2,000
1,000
0
10
20
30
40
2Theta (Coupled TwoTheta/Theta) WL = 1.54060

**FIG. 1**

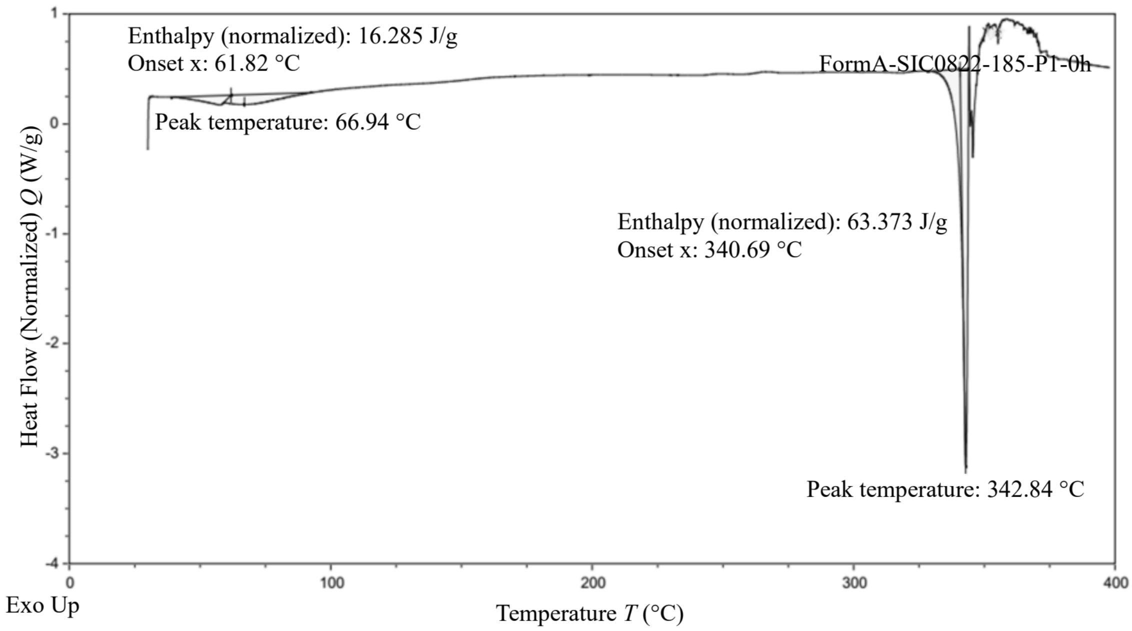
Enthalpy (normalized): 16.285 J/g
Onset x: 61.82 °C
Peak temperature: 66.94 °C
FormA-SIC0822-185-P1-0h
Enthalpy (normalized): 63.373 J/g
Onset x: 340.69 °C
Peak temperature: 342.84 °C
Heat Flow (Normalized) Q (W/g)
1
0
-1
-2
-3
-4
0
100
200
300
400
Exo Up
Temperature T (°C)

**FIG. 2**

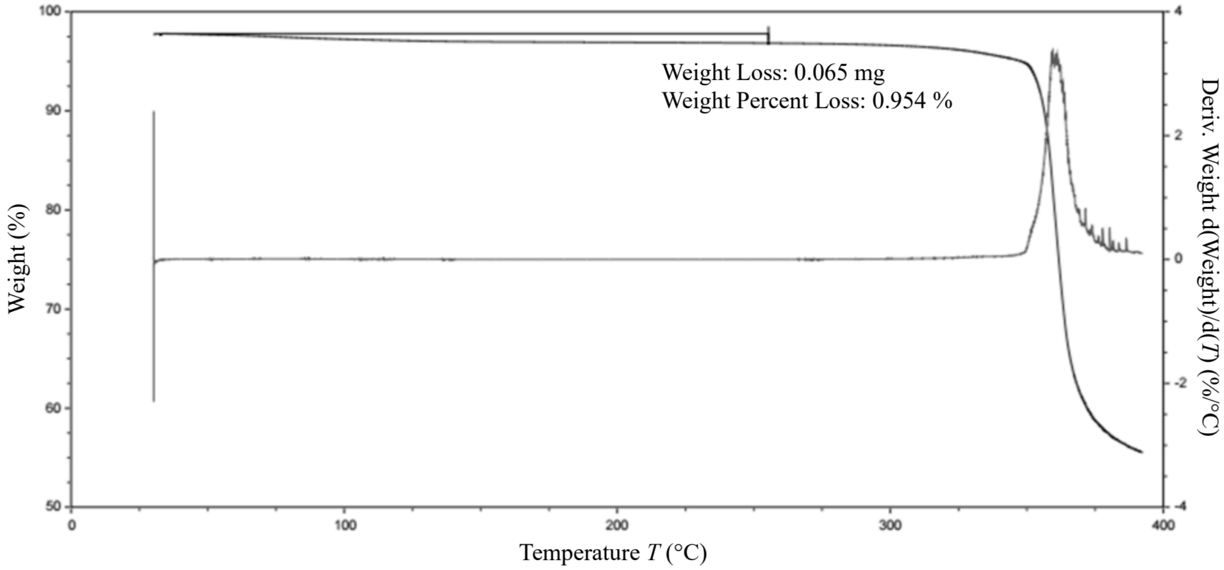
Weight (%)
Deriv. Weight d(Weight)/d(T) (%/°C)
Temperature T (°C)
Weight Loss: 0.065 mg
Weight Percent Loss: 0.954 %

**FIG. 3**

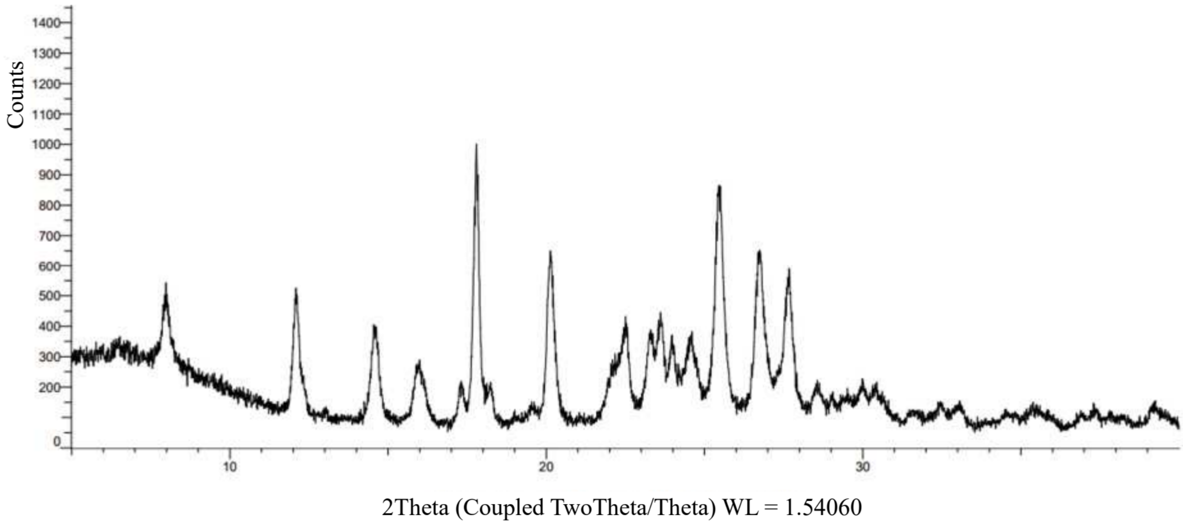
Counts
2Theta (Coupled TwoTheta/Theta) WL = 1.54060

**FIG. 4**

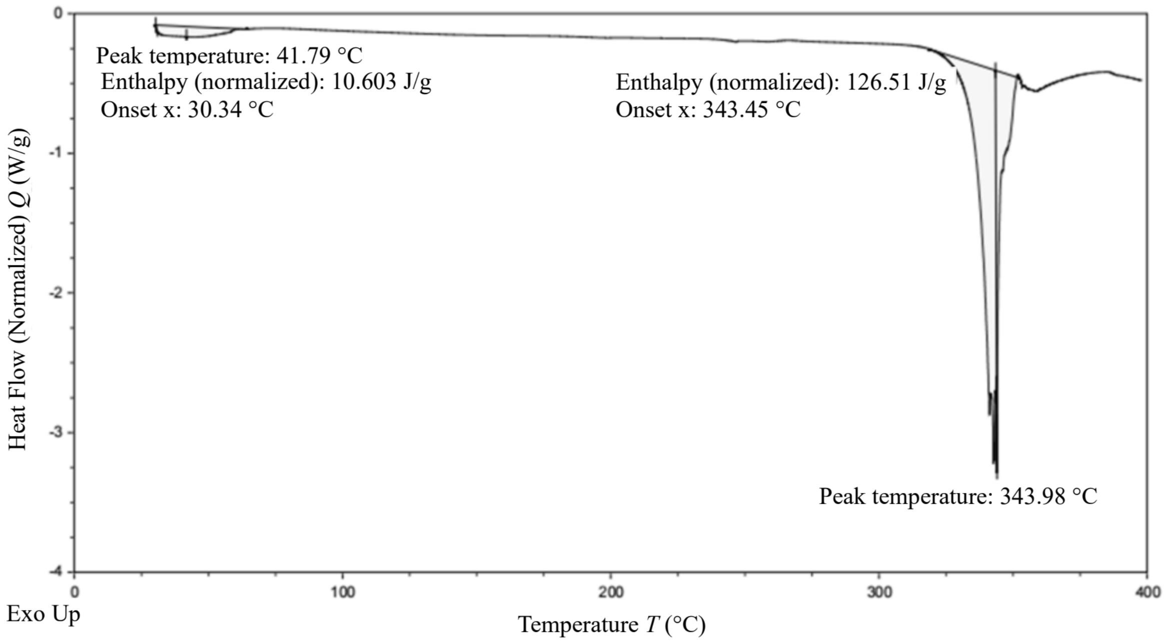
Peak temperature: 41.79 °C
Enthalpy (normalized): 10.603 J/g
Onset x: 30.34 °C
Enthalpy (normalized): 126.51 J/g
Onset x: 343.45 °C
Peak temperature: 343.98 °C
Heat Flow (Normalized) Q (W/g)
0
-1
-2
-3
-4
0
100
200
300
400
Exo Up
Temperature T (°C)

**FIG. 5**

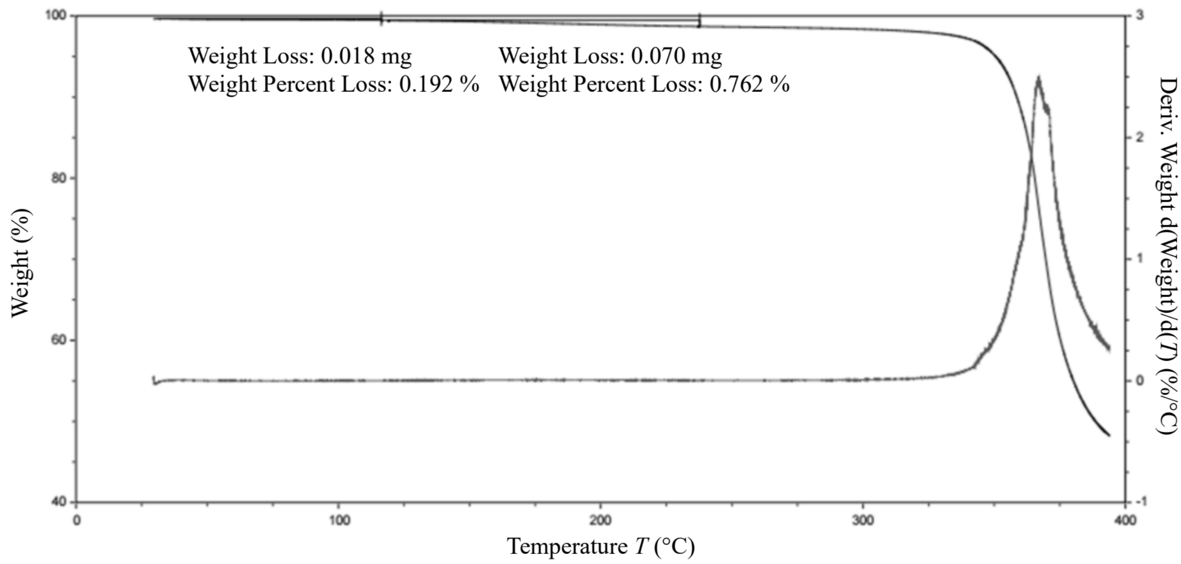
Weight Loss: 0.018 mg
Weight Percent Loss: 0.192 %
Weight Loss: 0.070 mg
Weight Percent Loss: 0.762 %
Weight (%)
100
80
60
40
Deriv. Weight d(Weight)/d(T) (%/°C)
3
2
1
0
-1
0
100
200
300
400
Temperature T (°C)

**FIG. 6**

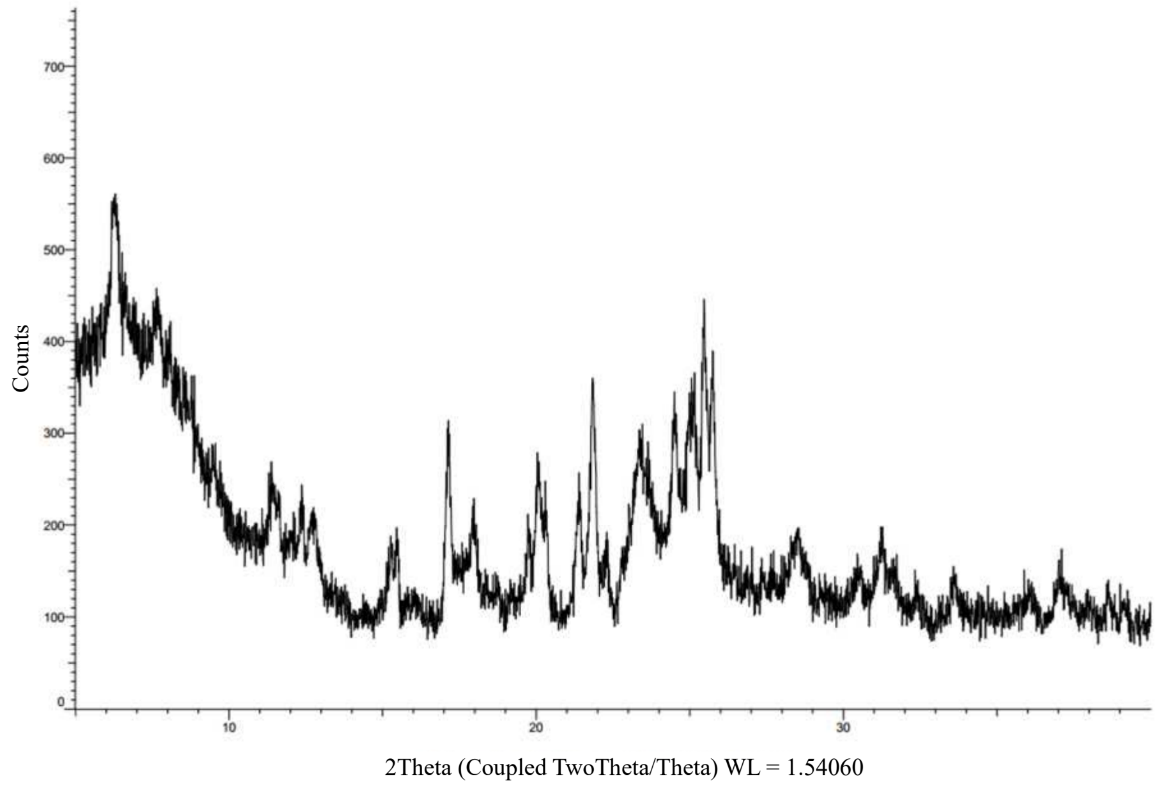
Counts
700
600
500
400
300
200
100
0
10
20
30
2Theta (Coupled TwoTheta/Theta) WL = 1.54060

**FIG. 7**

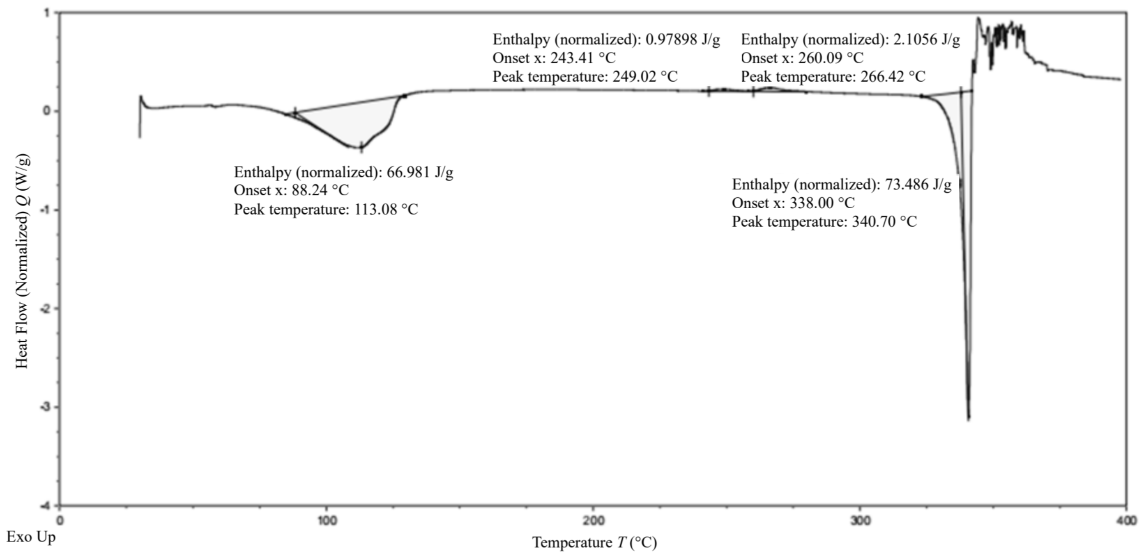
Enthalpy (normalized): 0.97898 J/g
Onset x: 243.41 °C
Peak temperature: 249.02 °C
Enthalpy (normalized): 2.1056 J/g
Onset x: 260.09 °C
Peak temperature: 266.42 °C
Enthalpy (normalized): 66.981 J/g
Onset x: 88.24 °C
Peak temperature: 113.08 °C
Enthalpy (normalized): 73.486 J/g
Onset x: 338.00 °C
Peak temperature: 340.70 °C
Heat Flow (Normalized) Q (W/g)
1
0
-1
-2
-3
-4
0
100
200
300
400
Exo Up
Temperature T (°C)

**FIG. 8**

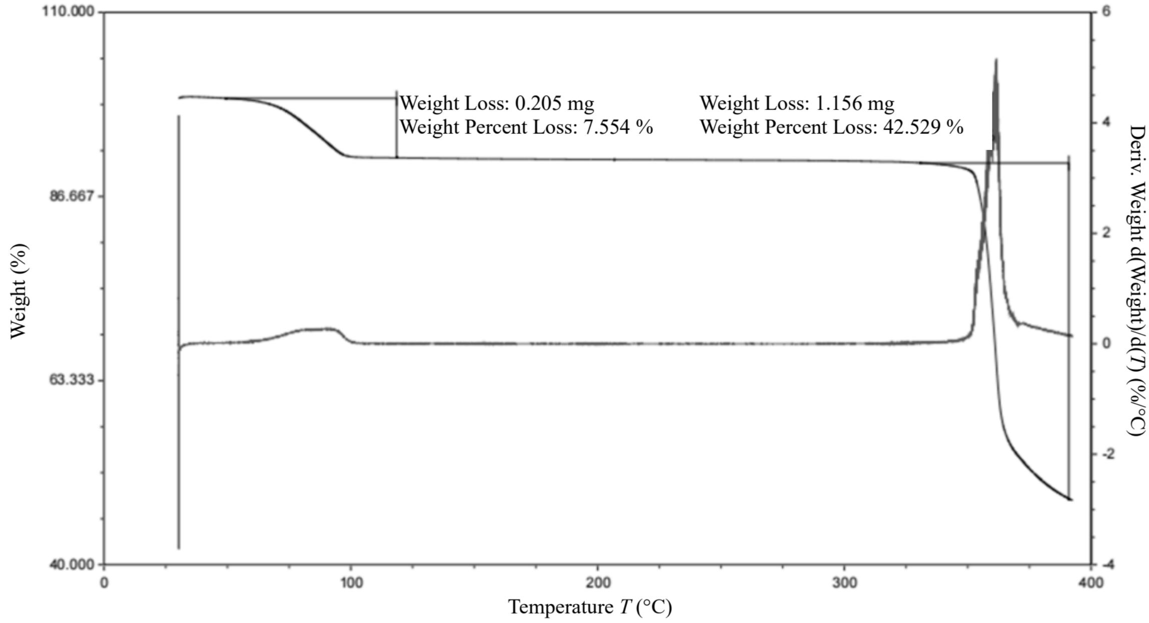


FIG. 9

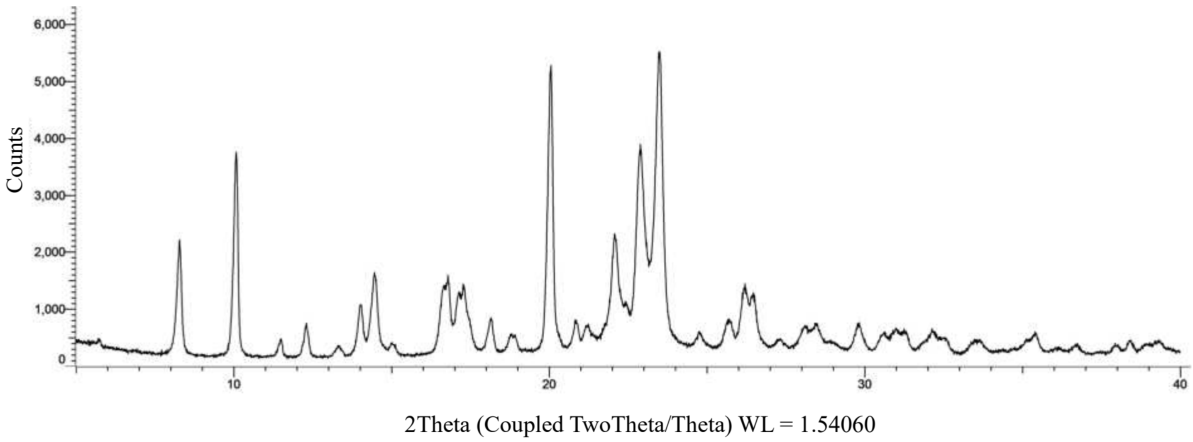


FIG. 10

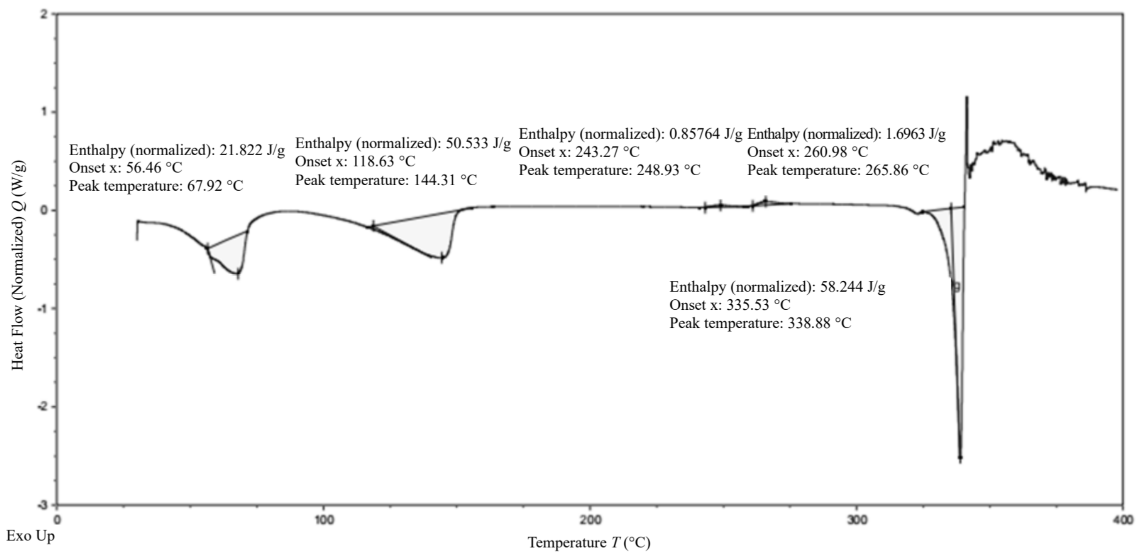


FIG. 11

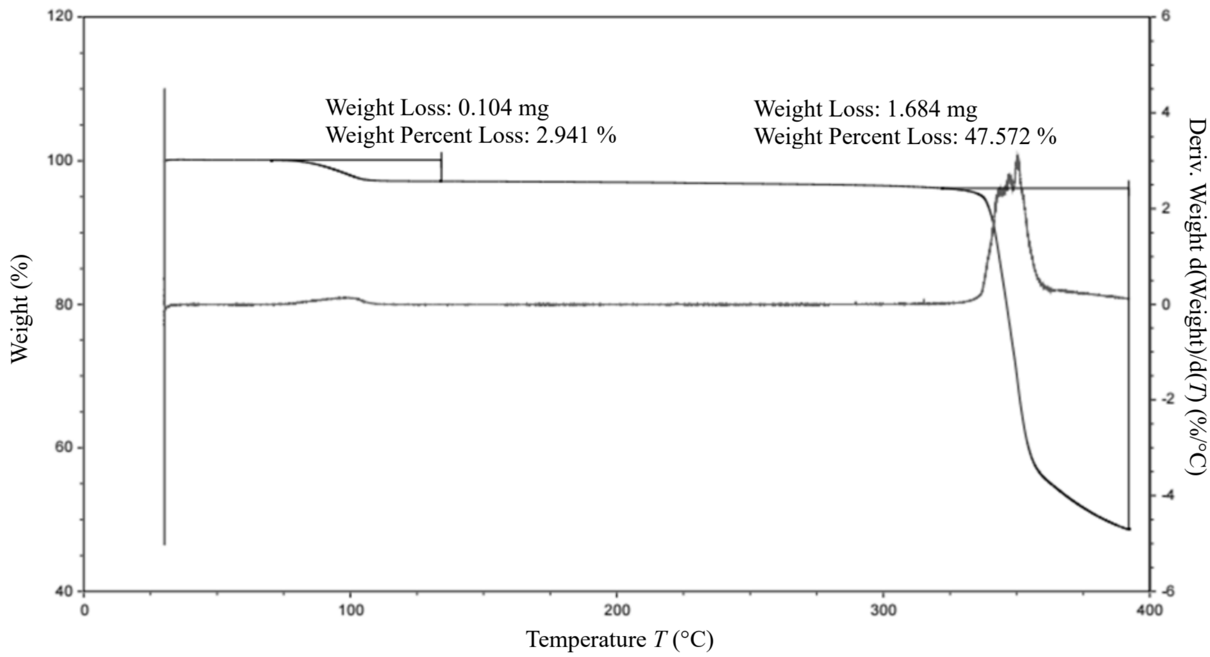
Weight Loss: 0.104 mg
Weight Percent Loss: 2.941 %
Weight Loss: 1.684 mg
Weight Percent Loss: 47.572 %
Weight (%)
Deriv. Weight d(Weight)/d(T) (%/°C)
Temperature T (°C)

**FIG. 12**

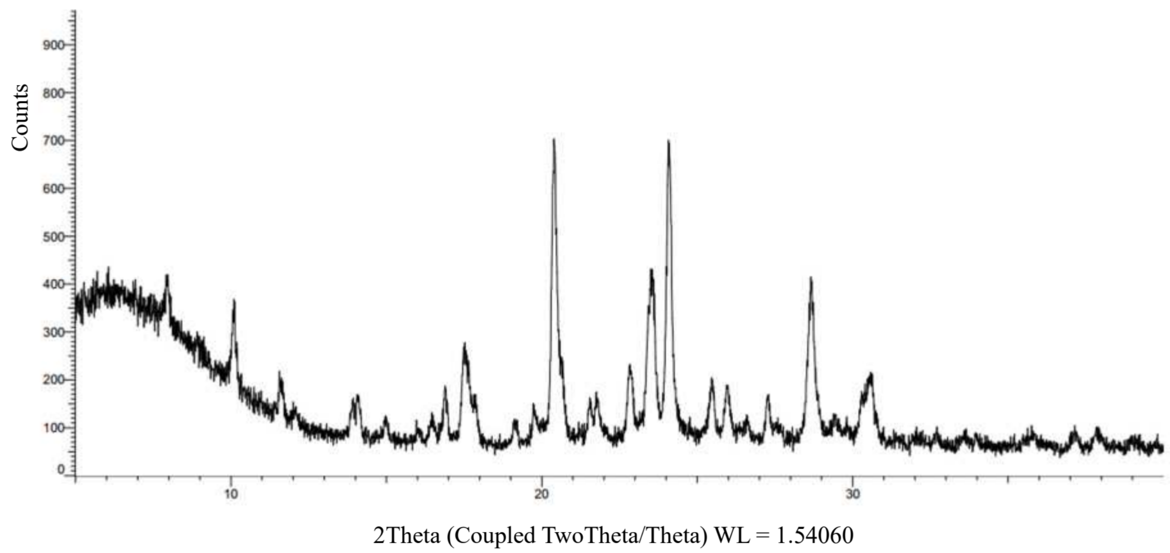
Counts
2Theta (Coupled TwoTheta/Theta) WL = 1.54060

**FIG. 13**

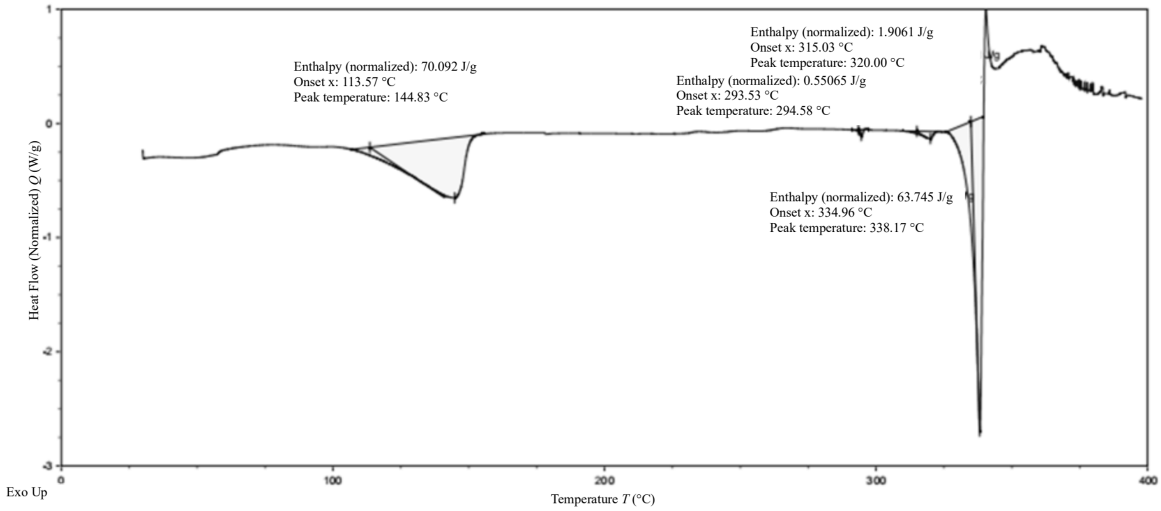
Enthalpy (normalized): 70.092 J/g
Onset x: 113.57 °C
Peak temperature: 144.83 °C
Enthalpy (normalized): 1.9061 J/g
Onset x: 315.03 °C
Peak temperature: 320.00 °C
Enthalpy (normalized): 0.55065 J/g
Onset x: 293.53 °C
Peak temperature: 294.58 °C
Enthalpy (normalized): 63.745 J/g
Onset x: 334.96 °C
Peak temperature: 338.17 °C
Heat Flow (Normalized) Q (W/g)
Exo Up
Temperature T (°C)

**FIG. 14**

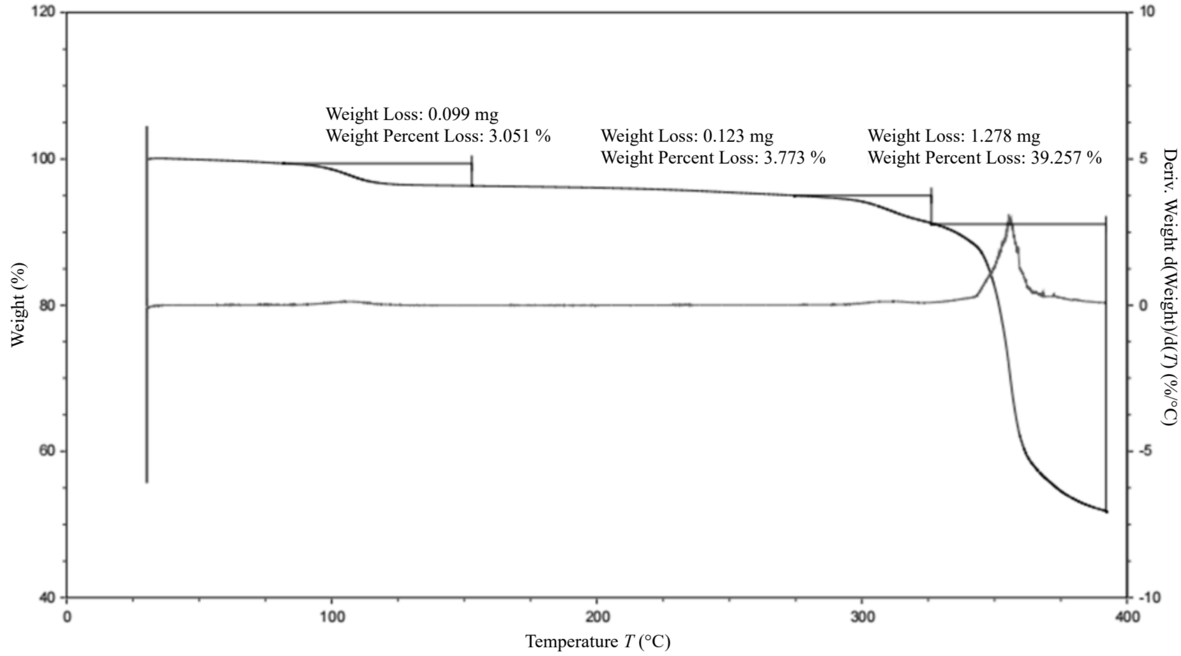
Weight Loss: 0.099 mg
Weight Percent Loss: 3.051 %
Weight Loss: 0.123 mg
Weight Percent Loss: 3.773 %
Weight Loss: 1.278 mg
Weight Percent Loss: 39.257 %
Weight (%)
Deriv. Weight d(Weight)/d(T) (%/°C)
Temperature T (°C)

**FIG. 15**

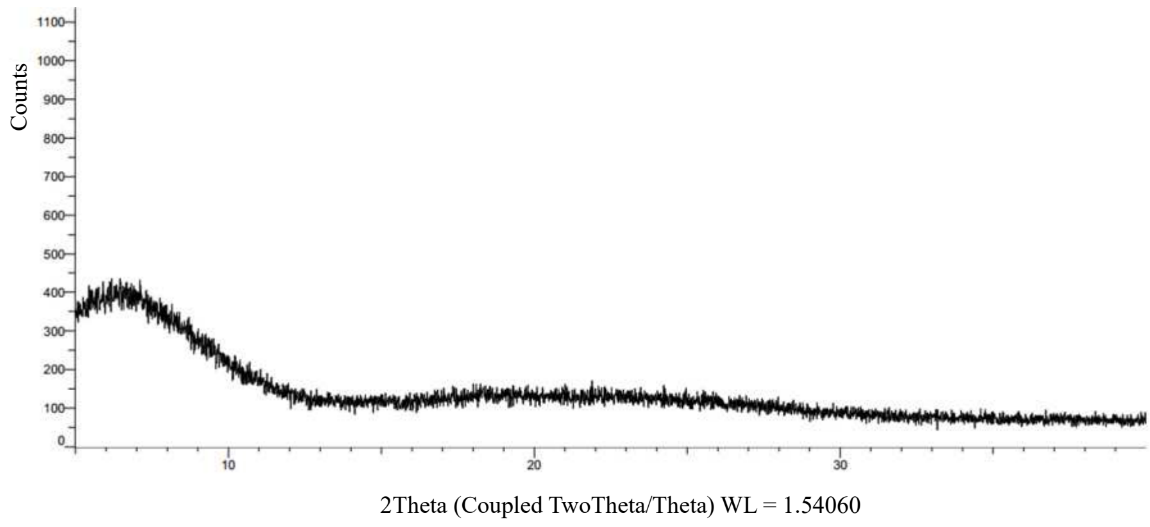
Counts
1100
1000
900
800
700
600
500
400
300
200
100
0
10
20
30
2Theta (Coupled TwoTheta/Theta) WL = 1.54060

FIG. 16

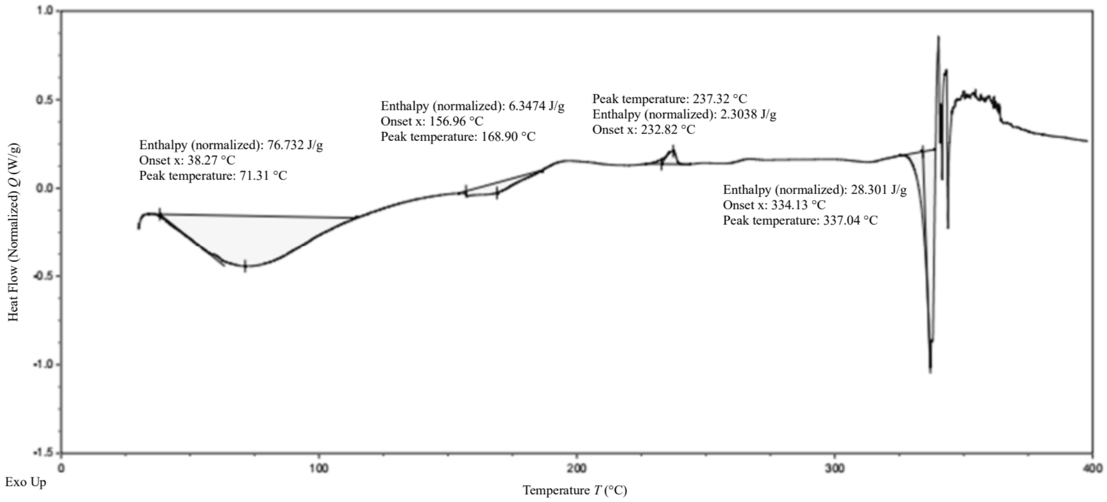
Heat Flow (Normalized) Q (W/g)
1.0
0.5
0.0
-0.5
-1.0
-1.5
0
100
200
300
400
Exo Up
Temperature T (°C)
Enthalpy (normalized): 76.732 J/g
Onset x: 38.27 °C
Peak temperature: 71.31 °C
Enthalpy (normalized): 6.3474 J/g
Onset x: 156.96 °C
Peak temperature: 168.90 °C
Peak temperature: 237.32 °C
Enthalpy (normalized): 2.3038 J/g
Onset x: 232.82 °C
Enthalpy (normalized): 28.301 J/g
Onset x: 334.13 °C
Peak temperature: 337.04 °C

FIG. 17

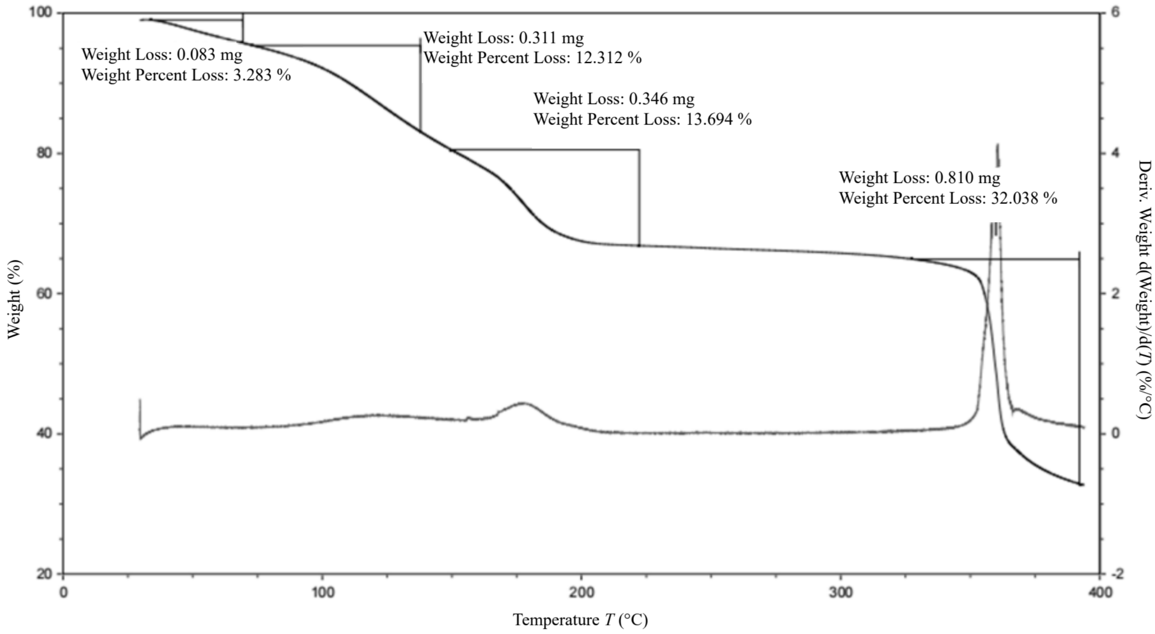
Weight Loss: 0.083 mg
Weight Percent Loss: 3.283 %
Weight Loss: 0.311 mg
Weight Percent Loss: 12.312 %
Weight Loss: 0.346 mg
Weight Percent Loss: 13.694 %
Weight Loss: 0.810 mg
Weight Percent Loss: 32.038 %
Weight (%)
Deriv. Weight d(Weight)/d(T) (%/°C)
Temperature T (°C)
100
80
60
40
20
0
100
200
300
400
6
4
2
0
-2

**FIG. 18**

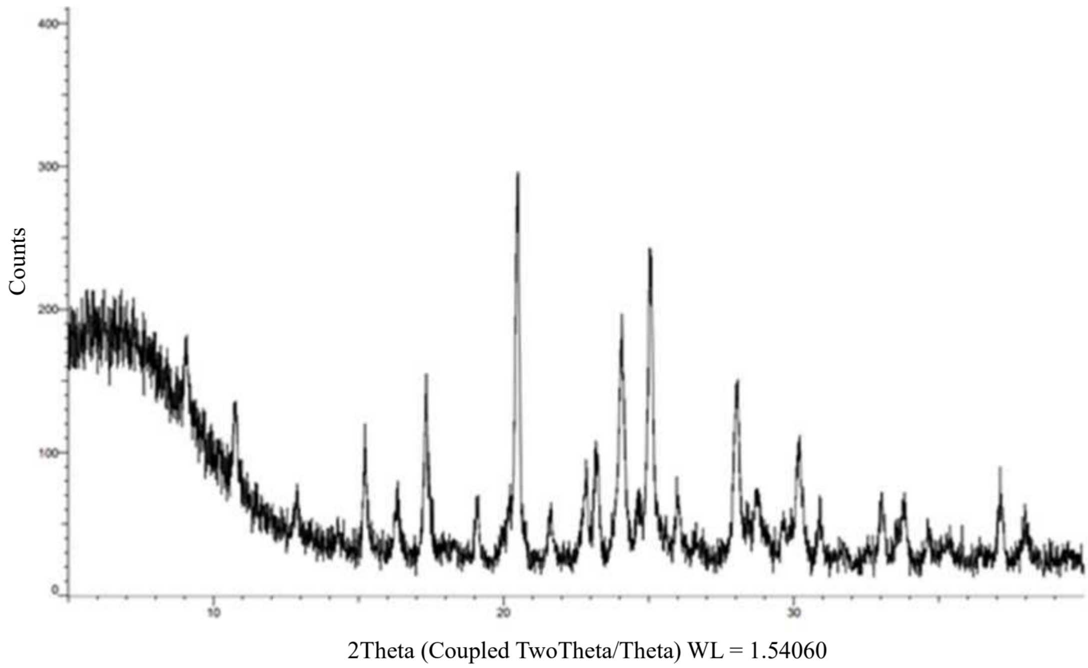
Counts
400
300
200
100
0
10
20
30
2Theta (Coupled TwoTheta/Theta) WL = 1.54060

**FIG. 19**

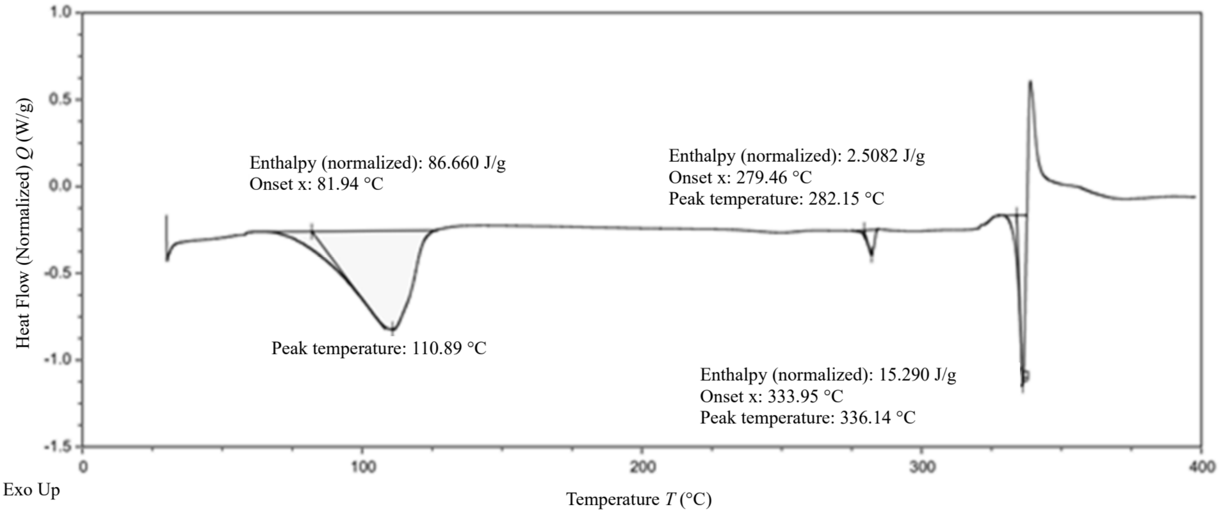
Heat Flow (Normalized) Q (W/g)
1.0
0.5
0.0
-0.5
-1.0
-1.5
Enthalpy (normalized): 86.660 J/g
Onset x: 81.94 °C
Peak temperature: 110.89 °C
Enthalpy (normalized): 2.5082 J/g
Onset x: 279.46 °C
Peak temperature: 282.15 °C
Enthalpy (normalized): 15.290 J/g
Onset x: 333.95 °C
Peak temperature: 336.14 °C
0
100
200
300
400
Exo Up
Temperature T (°C)

FIG. 20

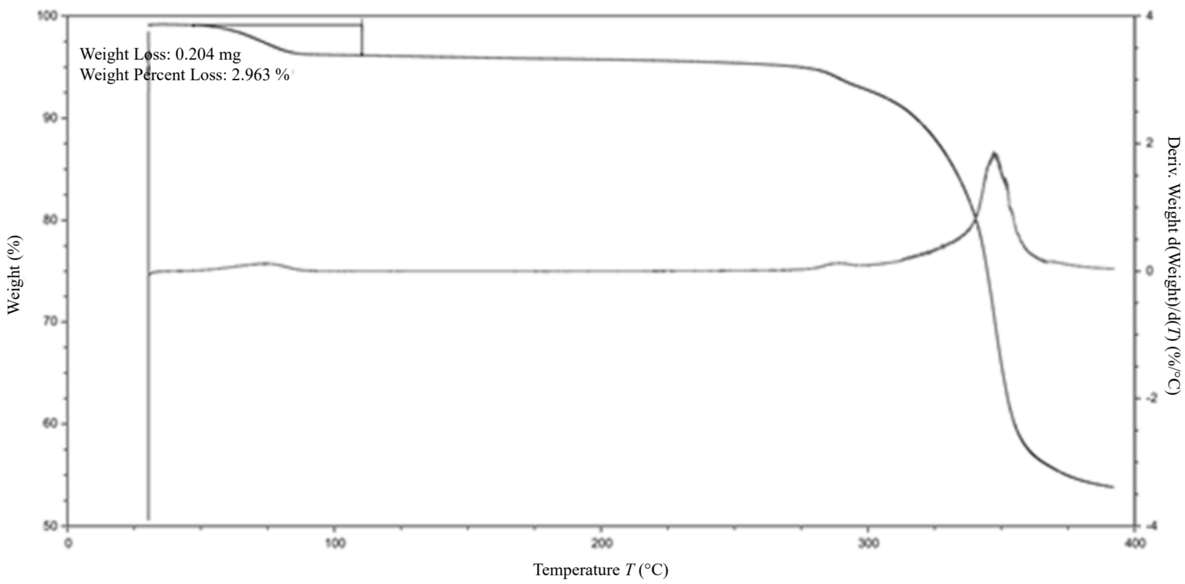
Weight (%)
100
90
80
70
60
50
Weight Loss: 0.204 mg
Weight Percent Loss: 2.963 %
Deriv. Weight d(Weight)/d(T) (%/°C)
4
2
0
-2
-4
0
100
200
300
400
Temperature T (°C)

FIG. 21

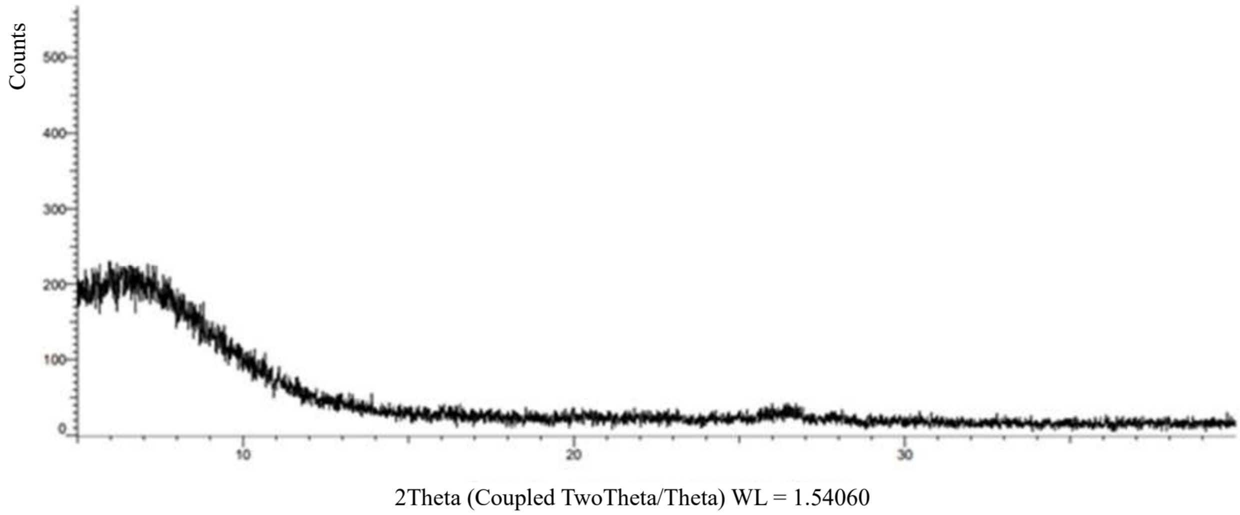
Counts
500
400
300
200
100
0
10
20
30
2Theta (Coupled TwoTheta/Theta) WL = 1.54060

**FIG. 22**

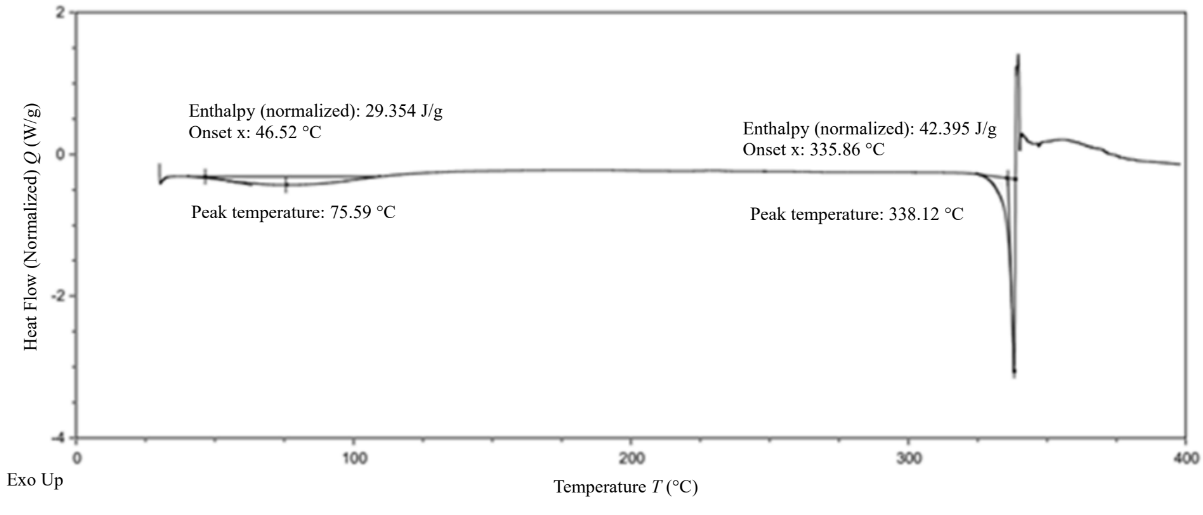
Enthalpy (normalized): 29.354 J/g
Onset x: 46.52 °C
Peak temperature: 75.59 °C
Enthalpy (normalized): 42.395 J/g
Onset x: 335.86 °C
Peak temperature: 338.12 °C
Heat Flow (Normalized) Q (W/g)
2
0
-2
-4
0
100
200
300
400
Exo Up
Temperature T (°C)

**FIG. 23**

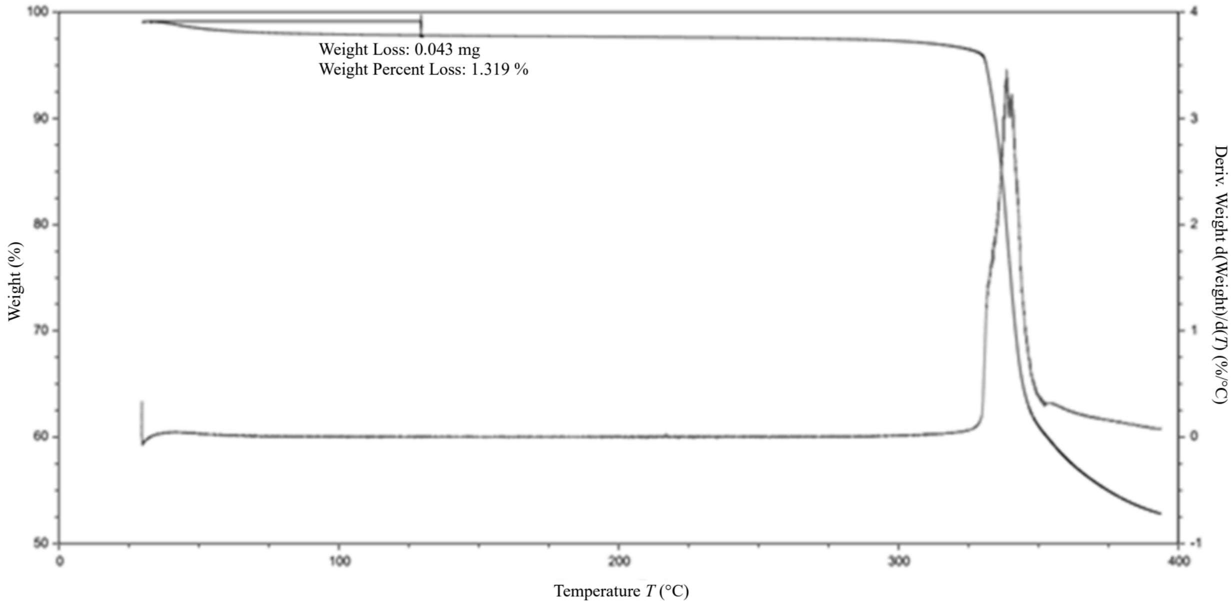
Weight Loss: 0.043 mg
Weight Percent Loss: 1.319 %
100
90
80
70
60
50
Weight (%)
4
3
2
1
0
-1
Deriv. Weight d(Weight)/d(T) (%/°C)
0
100
200
300
400
Temperature T (°C)

FIG. 24

## INTERNATIONAL SEARCH REPORT

International application No.
**PCT/CN2024/104980**

**A. CLASSIFICATION OF SUBJECT MATTER**

C07D513/04(2006.01)i; C07D401/14(2006.01)i; A61K31/4985(2006.01)i; A61K31/429(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, ENTXT, CNKI, ISI Web of Knowledge, CAPLUS(STN), REGISTRY(STN): 噻唑, 吡嗪, POLQ, DNA聚合酶, 晶型, 晶体, 水合物, 溶剂合物, thiazole, piperazin, cryst+, solvent

**C. DOCUMENTS CONSIDERED TO BE RELEVANT**

| Category* | Citation of document, with indication, where appropriate, of the relevant passages | Relevant to claim No. |
|---|---|---|
| PX | WO 2023134739 A1 (NANJING ZAIMING PHARMACEUTICAL CO., LTD.) 20 July 2023 (2023-07-20) description, page 9, last paragraph, page 10, line 8, first compound, and page 13, lines 7-13, and embodiment 23 | 1-36 |
| A | WO 2020160213 A1 (IDEAYA BIOSCIENCES, INC.) 06 August 2020 (2020-08-06) description, paragraphs [0009], [0080], [0086]-[0087] and [0093]-[0094] | 1-36 |
| A | CN 114127062 A (IDEAYA BIOSCIENCES, INC.) 01 March 2022 (2022-03-01) claims 1-72, and description, paragraphs [0239]-[0263] | 1-36 |

☐ Further documents are listed in the continuation of Box C.　　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 September 2024** | **08 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/104980**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **33, 35**
because they relate to subject matter not required to be searched by this Authority, namely:

Claims 33 and 35 relate to a method for preventing or treating diseases. The present search report is provided on the basis of the use of a compound or a pharmaceutical composition thereof in the preparation of a drug for treating diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2024/104980**

| Patent document cited in search report | Publication date (day/month/year) | Patent family member(s) | Publication date (day/month/year) |
|---|---|---|---|
| WO 2023134739 A1 | 20 July 2023 | AU 2023207390 A1 | 01 August 2024 |
| | | TW 202334169 A | 01 September 2023 |
| WO 2020160213 A1 | 06 August 2020 | EP 3917919 A1 | 08 December 2021 |
| | | AU 2020215042 A1 | 19 August 2021 |
| | | BR 112021015007 A2 | 05 October 2021 |
| | | CA 3127642 A1 | 06 August 2020 |
| | | US 2021387968 A1 | 16 December 2021 |
| | | JP 2022519535 A | 24 March 2022 |
| CN 114127062 A | 01 March 2022 | TW 202110834 A | 16 March 2021 |
| | | WO 2020243459 A1 | 03 December 2020 |
| | | CA 3141134 A1 | 03 December 2020 |
| | | US 2023078112 A1 | 16 March 2023 |
| | | JP 2022535227 A | 05 August 2022 |
| | | AU 2020282768 A1 | 23 December 2021 |
| | | AU 2020282768 B2 | 14 September 2023 |
| | | ES 2968796 T3 | 14 May 2024 |
| | | EP 3976608 A1 | 06 April 2022 |
| | | EP 3976608 C0 | 08 November 2023 |
| | | BR 112021024101 A2 | 08 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310852344

### Non-patent literature cited in the description


- **KENT et al.** *Nature Structural & Molecular Biology*, 2015, vol. 22 (3), 230-237
- **MATEOS-GOMEZ et al.** *Nature*, 2015, vol. 518 (7538), 254-257
- **WOOD** ; **DOUBLIE**. *DNA Repair*, 2016, vol. 44, 22-32
- **CECCALDI et al.** *Nature*, 2015, vol. 518 (7538), 258-262
- **HIGGINS et al.** *Oncotarget*, 2010, vol. 1, 175-184
- **LEMEE et al.** *PNAS*, 2010, vol. 107 (30), 13390-13395
- **KAWAMURA et al.** *International Journal of Cancer*, 2004, vol. 109 (1), 9-16